# EUROPEAN PATENT SPECIFICATION

(11) **EP 4 673 053 B1**
(45) Date of publication and mention of the grant of the patent: **08.04.2026**
(21) Application number: 24704515.6
(22) Date of filing: 19.02.2024
(51) Int. Cl.: A61B 6/04, A61B 6/00, G06T 7/73, G06N 3/02

(54) **PROVIDING POSE ADJUSTMENT INFORMATION**
BEREITSTELLUNG VON POSENEINSTELLUNGSINFORMATIONEN
FOURNITURE D'INFORMATIONS D'AJUSTEMENT DE POSE

(30) Priority: 28.02.2023 EP 23159085
(43) Date of publication of application: 07.01.2026
(73) Proprietor: Koninklijke Philips N.V., 5656 AG Eindhoven (NL)
(72) Inventor: YOUNG, Stewart Matthew, 5656 AG Eindhoven (NL); BYSTROV, Daniel, 5656 AG Eindhoven (NL); KROENKE-HILLE, Sven, 5656 AG Eindhoven (NL); VON BERG, Jens, 5656 AG Eindhoven (NL); BRUECK, Heiner Matthias, 5656 AG Eindhoven (NL); SENEGAS, Julien Thomas, 5656 AG Eindhoven (NL); GOOSSEN, Andre, 5656 AG Eindhoven (NL); MENSER, Bernd, 5656 AG Eindhoven (NL); HARDER, Tim Philipp, 5656 AG Eindhoven (NL)
(74) Representative: Philips Intellectual Property & Standards
(86) International application number: PCT/EP2024/054146
(87) International publication number: WO 2024/179871

(56) References cited:
- WO-A1-2022/099068
- DE-A1- 102020 213 690
- US-A1- 2018 116 613

## Description

### FIELD OF THE INVENTION

The present disclosure relates to providing pose adjustment information for adjusting a pose of an anatomical structure with respect to a projection X-ray imaging system in order to acquire a target X-ray projection image. A computer-implemented method, a computer program product, and a system, are disclosed.

### BACKGROUND OF THE INVENTION

Projection X-ray imaging systems include an X-ray source and an X-ray detector. The X-ray source and the X-ray detector are separated by an examination region. During an imaging operation, an anatomical structure such as an ankle, a leg or a chest of a subject, is disposed in the examination region in order to generate X-ray projection images representing the anatomical structure.

The positioning of anatomical structures in order to acquire X-ray projection images is conventionally performed based on the experience of an operator. The operator may position the patient by eye, sometimes via a monitor that displays a visible camera image of the object. The operator uses their experience to adjust the pose, i.e. the position and orientation, of the anatomical structure, in order to try to provide a requested image of the anatomical structure. Markings on the X-ray detector that indicate the extent of an X-ray detector's radiation-sensitive region are used to ensure that the anatomical structure is correctly positioned with respect to the X-ray detector prior to acquiring the image.

However, conventional approaches to the positioning of anatomical structures in order to acquire X-ray projection images have drawbacks. Often, a physician requesting the X-ray projection image desires an X-ray projection image in which features of the anatomical structure such as the bones are arranged in a specific manner, or an image in which the "joint gap" between bones is visible. Obtaining the desired level of positioning accuracy can be difficult for the operator. Any deficiencies in the image may result in the need to re-take the image. In some situations a deficiency in the image is noted by the operator and the re-take can therefore be performed with minimal delay. In other situations, a deficiency in the image may only be noted by the requesting physician at a later point in time, resulting in the need to re-call the subject in order to acquire a follow-up image. Both situations hamper workflow, and increase the amount of X-ray radiation dose to the subject.

A document WO 2019/134874 A1 discloses that the appropriate positioning of a patient in an X-ray imaging system can present difficulties for medical professional owing, on one hand to the small size of important anatomical aspects which need to be captured in X-ray images, and on the other hand to the significant movements in a field of view presented by a typical patient. This document proposes to obtain an image of the position of a patient in the field of view at approximately the same time that an initial X-ray image is obtained. If it proves necessary to obtain a subsequent X-ray image with updated field of view settings (for example, collimation parameters), the movement of the patient at the point of taking the second image is factored into the provision of updated field of view settings. Another document DE 10 2020 213690 A1 discloses a method for adjusting the shape of a headrest, which involves calculating a target pose of the head of the patient based on the radiological image data relating to the head of the patient. A landmark actual position which relates to a set of landmarks is determined based on optical image data acquired by a camera.

However, there is a need for improvements to facilitate improved positioning of anatomical structures when acquiring X-ray projection images.

It is to be noted that US patent application publication US 2018/247427 A1 discloses a method for patient positioning in which a depth camera image is used to determine a first patient pose at a first moment in time, and in which a second pose is determined based on sensing of the patient on a bed of a medical scanner at a different moment in time from the first pose, and in which the two poses are compared.

It is further to be noted that PCT patent application publication WO 2020/056086 A1 discloses a intra-operative guidance system in which a plurality of fluoroscopic surgical images are used to calculate intra-operative surgical risks.

### SUMMARY OF THE INVENTION

The subject matter of the present invention is claimed in the independent claims. Advantageous embodiments of the present invention are claimed in the dependent claims.

According to one aspect of the present disclosure, a computer-implemented method of providing pose adjustment information for adjusting a pose of an anatomical structure with respect to a projection X-ray imaging system in order to acquire a target X-ray projection image representing the anatomical structure is provided. The method includes:
- receiving image data, the image data comprising:
- an initial X-ray projection image representing the anatomical structure, the initial X-ray projection image being acquired by the projection X-ray imaging system with the anatomical structure in an initial pose with respect the projection X-ray imaging system;
- an initial camera image representing the anatomical structure, the initial camera image being acquired by a camera configured to view the anatomical structure, the initial camera image being acquired with the anatomical structure in the initial pose with respect the projection X-ray imaging system; and

- a subsequent camera image representing the anatomical structure, the subsequent camera image being acquired by a camera configured to view the anatomical structure, the subsequent camera image being acquired with the anatomical structure in a subsequent pose with respect the projection X-ray imaging system and at a later point in time to the initial camera image;
- determining, based on the initial X-ray projection image, pose adjustment information for adjusting the initial pose of the anatomical structure with respect to the projection X-ray imaging system in order to acquire the target X-ray projection image;
- determining a registration between the anatomical structure in the initial X-ray projection image and the anatomical structure in the initial camera image;
- deforming the initial camera image using the registration and the pose adjustment information to provide a target camera image corresponding to the target X-ray projection image;
- determining the pose adjustment information for adjusting the subsequent pose of the anatomical structure with respect to the projection X-ray imaging system in order to acquire the target X-ray projection image, based on a deviation between the subsequent camera image and the target camera image; and
- outputting the pose adjustment information.

The method provides pose adjustment information that may be used to adjust an initial pose of an anatomical structure with respect to the projection X-ray imaging system in order to acquire a target X-ray projection image representing the anatomical structure, or in other words, a desired image. An initial X-ray projection image, and an initial camera image, are acquired with the anatomical structure in the initial pose. The initial X-ray projection image may deviate from the target X-ray projection image. Pose adjustment information is then generated to determine how the anatomical structure in the initial X-ray projection image should be adjusted in order to acquire the target X-ray projection image. This operation may be performed using a neural network.

The registration operation and the deformation operation, are then used in combination with the pose adjustment information to provide a target camera image, i.e. an image showing how the anatomical structure should have appeared if the target X-ray projection image had been acquired. At a later point in time, a subsequent camera image is acquired with the anatomical structure in a subsequent pose. A deviation between the subsequent camera image and the target camera image is used to provide pose adjustment information for acquiring the target X-ray projection image. Since, in this method, the pose adjustment information is generated using the deviation between the subsequent camera image and the target camera image, the target X-ray projection image is acquired by positioning the anatomical structure without the need to acquire a subsequent X-ray projection image. This has the advantage of providing the target X-ray projection image with reduced X-ray dose to the subject.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating a first perspective of an arrangement including an X-ray imaging system 130a, 130b, and an anatomical structure 120, in accordance with some aspects of the present disclosure.
Fig. 2 is a schematic diagram illustrating a second perspective of an arrangement including an X-ray imaging system 130a, 130b, and an anatomical structure 120, in accordance with some aspects of the present disclosure.
Fig. 3 is a schematic diagram illustrating a first example of an anatomical structure 120 in an initial pose, α₁, with respect to a projection X-ray imaging system 130b, in accordance with some aspects of the present disclosure.
Fig. 4 is a schematic diagram illustrating a first example of an anatomical structure 120 in a target pose, α_{T}, with respect the projection X-ray imaging system 130b, in accordance with some aspects of the present disclosure.
Fig. 5 is a schematic diagram illustrating a second example of an anatomical structure 120 in an initial pose, ϕ₁, with respect to a projection X-ray imaging system 130b, in accordance with some aspects of the present disclosure.
Fig. 6 is a schematic diagram illustrating a second example of an anatomical structure 120 in a target pose, ϕ_{T}, with respect to a projection X-ray imaging system 130b, in accordance with some aspects of the present disclosure.
Fig. 7 is a flowchart illustrating an example of a method of providing pose adjustment information for adjusting a pose of an anatomical structure with respect to a projection X-ray imaging system in order to acquire a target X-ray projection image of the anatomical structure, in accordance with some aspects of the present disclosure.
Fig. 8 is a flowchart illustrating an example of a method of providing pose adjustment information for adjusting a pose of an anatomical structure with respect to a projection X-ray imaging system in order to acquire a target X-ray projection image representing the anatomical structure, in accordance with some aspects of the present disclosure.
Fig. 9 is a schematic diagram illustrating an example of a method of providing pose adjustment information 310 for adjusting a pose α₂ of an anatomical structure 120 with respect to a projection X-ray imaging system in order to acquire a target X-ray projection image 310_{T} representing the anatomical structure 120, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to an X-ray imaging system, may be implemented in a computer implemented method, and in a computer program product, in a corresponding manner.

In the following description, reference is made to a projection X-ray imaging system. An example of a current projection X-ray imaging system that may serve as the projection X-ray imaging system is the DigitalDiagnost C90 marketed by Philips Healthcare, Best, the Netherlands. Other projection X-ray imaging systems may also serve as the projection X-ray imaging system. In some example arrangements described herein, projection X-ray imaging systems are referred-to wherein an X-ray source is mounted to a ceiling via a gantry, and a corresponding X-ray detector is mounted to a stand and held in the vertical position. However, the principles disclosed herein are not limited to this particular arrangement, and it is to be appreciated that other arrangements may alternatively be used wherein the X-ray source and X-ray detector are mounted, or supported, in a different manner, and in different positions.

Reference is also made herein to examples in which the projection X-ray imaging system is used to generate X-ray projection images representing an anatomical structure in the form of an ankle. However, it is to be appreciated that the anatomical structure may alternatively be any anatomical region, including for example the leg, arm, chest, and so forth.

In the following description, reference is made to various methods that are implemented by a processor, i.e. a computer. It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, there is a need for improvements to facilitate improved positioning of anatomical structures when acquiring X-ray projection images.

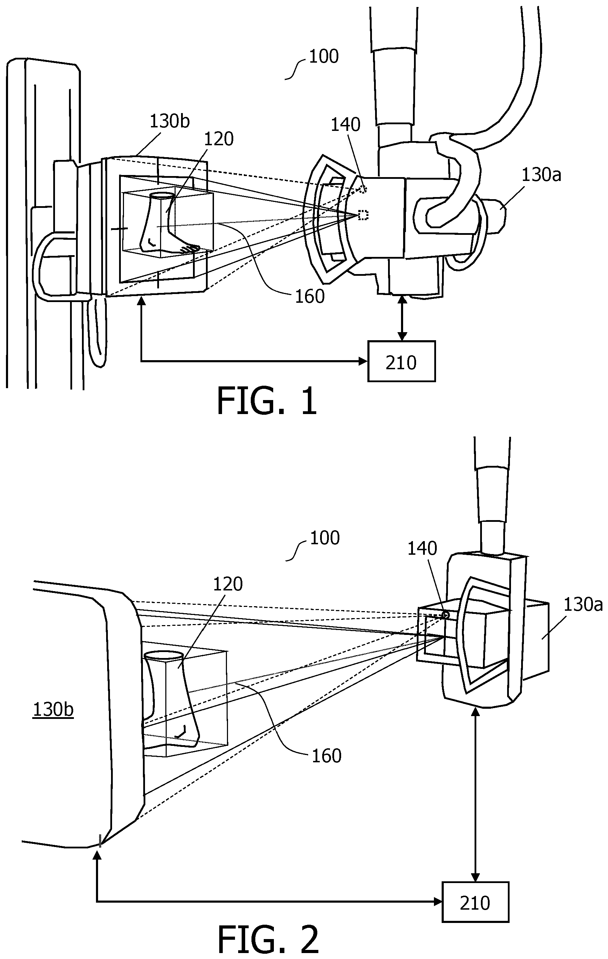

Fig. 1 is a schematic diagram illustrating a first perspective of an arrangement including an X-ray imaging system 130a, 130b, and an anatomical structure 120, in accordance with some aspects of the present disclosure. The X-ray imaging system illustrated in Fig. 1 includes an X-ray source 130a, and an X-ray detector 130b. The X-ray source 130a and the X-ray detector 130b are separated by an examination region. An anatomical structure 120 such as the ankle illustrated in Fig. 1 is disposed in the examination region in order to generate X-ray projection images representing the ankle. In the illustrated example, the X-ray source 130a is mounted to the ceiling via a gantry, and the X-ray detector 130b is mounted to a stand and held in the vertical position. Alternative arrangements, mounting structures, supporting arrangements, and positions of the X-ray source 130a and the X-ray detector 130b may also be used.

The arrangement illustrated in Fig. 1 also includes a camera 140. The camera 140 is configured to view the anatomical structure 120. In the arrangement illustrated in Fig. 1, the minimum extent of the field of view of the camera is indicated by the dashed lines extending between the camera 140 and the X-ray detector 130a. The camera 140 acquires camera images representing the anatomical structure 120. The camera 140 may also view at least a portion of the projection X-ray imaging system. For instance, the camera 140 may also view at least a portion of the X-ray source 130a, or at least a portion of the X-ray detector 130b. Thus, the camera 140 may acquire camera images representing the anatomical structure and at least a portion of the projection X-ray imaging system 130a, 130b.

The use of various types of camera is contemplated for use as the camera 140. For instance, the camera 140 may be an optical camera configured to generate optical images, or a depth camera configured to acquire depth images. The optical images may represent a portion of the visible spectrum and/or a portion of the infrared spectrum. The camera 140 may also provide both optical images and depth images. In general, the depth images generated by a depth camera represent variations in the range between the depth camera and points on the surfaces of objects within the depth camera's field of view. With reference to Fig. 1, the depth camera may therefore generate depth camera images representing a three-dimensional shape of a surface of the anatomical structure 120.

If the camera 140 illustrated in Fig. 1 is provided by a depth camera, the depth camera may employ various known principles to generate depth images. For instance, the depth camera may employ a time-of-flight, or LIDAR principle, or a structured light principle, or a binocular stereo vision principle. In the time-of-flight, or LIDAR principle, the time taken for emitted light pulses to travel from the position of the camera to objects in a scene and back again, is used to generate depth camera image data representing the range to the objects. The Azure Kinect DK depth camera, and the Intel RealSense^{™} LiDAR Camera L515 are examples of depth cameras that employ this principle. In the structured light principle, an optical pattern is projected onto the surface of objects within a scene, and the disparity between the original projected pattern, and the pattern that is deformed by the surface of the objects is imaged by one or more cameras. In the binocular stereo vision principle, different views of a scene are used to compute a depth map of the scene.

In the example arrangement illustrated in Fig. 1, the camera 140 is mechanically coupled to the X-ray source 130a. However the camera 140 may alternatively be positioned elsewhere in order to view the anatomical structure 120. For instance, the camera may alternatively be mechanically coupled to a wall, or to a ceiling of a room in which the projection X-ray imaging system 130a, 130b is located, or it may be mechanically coupled to a floor-based stand. The camera may alternatively be mobile. In some examples, the camera may therefore be capable of being moved around a room in which the X-ray imaging system 130a, 130b is located. In each of these alternative arrangements, the camera is able to view an anatomical structure 120 disposed in the examination region.

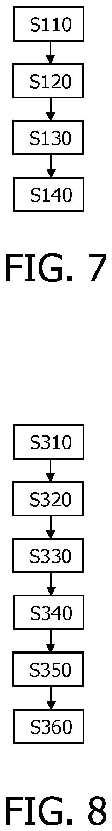

The arrangement illustrated in Fig. 1 also includes one or more processors 210. The one or more processors 210 perform operations relating to methods that are described below with reference to Fig. 7 and Fig. 8. The one or more processors 210 may also perform operations such as controlling the X-ray source 130a, and the X-ray detector 130b, in order to acquire X-ray projection images representing the anatomical structure 120. The one or more processors 210 may also perform operations such as controlling the camera 140 to generate camera images representing the anatomical structure.

Fig. 2 is a schematic diagram illustrating a second perspective of an arrangement including an X-ray imaging system 130a, 130b, and an anatomical structure 120, in accordance with some aspects of the present disclosure. The arrangement illustrated in Fig. 2 simply provides a different perspective of the same features that are illustrated in Fig. 1. Consequently a description of these features is not duplicated here for the sake of brevity.

In-use, it is desirable that the anatomical structure 120 illustrated in Fig. 1, and likewise in Fig. 2, is correctly positioned with respect to the projection X-ray imaging system 130a, 130b, in order to obtain a desired X-ray projection image of the anatomical structure 120.

In accordance with one aspect of the present disclosure, a system 100 is provided. The system includes one or more processors 210 that are configured to perform a method of providing pose adjustment information 110 for adjusting a pose of an anatomical structure 120 with respect to a projection X-ray imaging system 130a, 130b in order to acquire a target X-ray projection image representing the anatomical structure 120. The method comprises:
- receiving S110 image data, the image data comprising:
   - an initial X-ray projection image representing the anatomical structure 120, the initial X-ray projection image being acquired by the projection X-ray imaging system 130a, 130b with the anatomical structure 120 in an initial pose α₁, ϕ₁ with respect the projection X-ray imaging system 130a, 130b; and/or
   - a camera image representing the anatomical structure 120, the camera image being acquired by a camera 140 configured to view the anatomical structure 120, the camera image being acquired with the anatomical structure 120 in the initial pose α₁, ϕ₁ with respect the projection X-ray imaging system 130a, 130b;
   - inputting S120 the image data into a neural network; and
- in response to the inputting, generating S130 using the neural network, pose adjustment information 110 for adjusting the initial pose α₁, ϕ₁ of the anatomical structure 120 with respect to the projection X-ray imaging system 130a, 130b in order to acquire the target X-ray projection image; and
- outputting S140 the pose adjustment information 110; and
- wherein the neural network is trained to generate the pose adjustment information 110 using training data comprising a plurality of training images representing the anatomical structure 120, the training images respectively comprising X-ray projection images acquired with the anatomical structure in a current pose with respect the projection X-ray imaging system and/or camera images acquired by a camera configured to view the anatomical structure with the anatomical structure in the current pose with respect the projection X-ray imaging system, and wherein the training data further comprises, for each training image, corresponding pose adjustment information for adjusting the current pose of the anatomical structure with respect to the projection X-ray imaging system in order to acquire the target X-ray projection image.

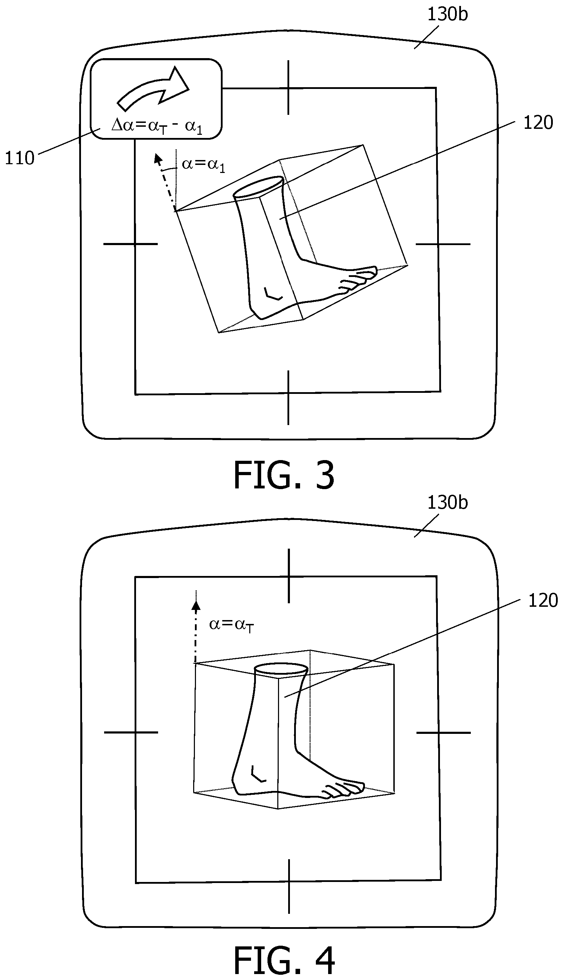

The above method provides pose adjustment information that can be used to adjust an initial pose of an anatomical structure with respect to the projection X-ray imaging system in order to acquire a target X-ray projection image representing the anatomical structure, or in other words, a desired image. Since, in the above method, the pose adjustment information is generated by a neural network, the pose adjustment information may be generated in a reliable manner. This has the advantage of reducing the number of re-takes of the image, thereby reducing X-ray dose to a subject and improving workflow. Examples of the above method are described with reference to Fig. 3 - Fig. 6, and with reference to Fig. 7, which is a flowchart illustrating an example of a method of providing pose adjustment information for adjusting a pose of an anatomical structure with respect to a projection X-ray imaging system in order to acquire a target X-ray projection image of the anatomical structure, in accordance with some aspects of the present disclosure.

With reference to the above method and the flowchart illustrated in Fig. 7, in the operation S110, image data is received. The image data that is received in the operation S110 includes an initial X-ray projection image representing the anatomical structure 120 and/or a camera image representing the anatomical structure 120. The initial X-ray projection image may be received from the projection X-ray imaging system 130a, 130b illustrated in Fig. 1, and the camera image may be received from the camera 140 illustrated in Fig. 1. In general, the image data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

The initial X-ray projection image is acquired by the projection X-ray imaging system 130a, 130b with the anatomical structure 120 in an initial pose α₁, ϕ₁ with respect the projection X-ray imaging system 130a, 130b. The camera image is acquired by a camera 140 configured to view the anatomical structure 120. The camera image is also acquired with the anatomical structure 120 in the initial pose α₁, ϕ₁ with respect the projection X-ray imaging system 130a, 130b.

The term pose as used herein refers to a position and/or an orientation of an object. In general, a pose of an object may be defined with respect to any reference coordinate system. For instance, the pose of an object may be expressed with respect to a reference coordinate system by means of a six-degrees of freedom "6-DOF" model that includes three degrees of freedom in translation with respect to three mutually orthogonal axes, and three degrees of freedom around the axes. With reference to the example illustrated in Fig. 1, it is useful to define the pose of the anatomical structure 120 with respect to the projection X-ray imaging system 130a, 130b because the image features in X-ray projection images that are acquired by the projection X-ray imaging system depend on the pose of the anatomical structure 120 with respect the projection X-ray imaging system.

Fig. 3 is a schematic diagram illustrating a first example of an anatomical structure 120 in an initial pose, α₁, with respect to a projection X-ray imaging system 130b, in accordance with some aspects of the present disclosure. In the example illustrated in Fig. 3, the pose is defined by the parameter α. The parameter α represents an angle between the Tibia within the ankle, i.e. the anatomical structure 120, and a vertical orientation of the X-ray detector 130b.

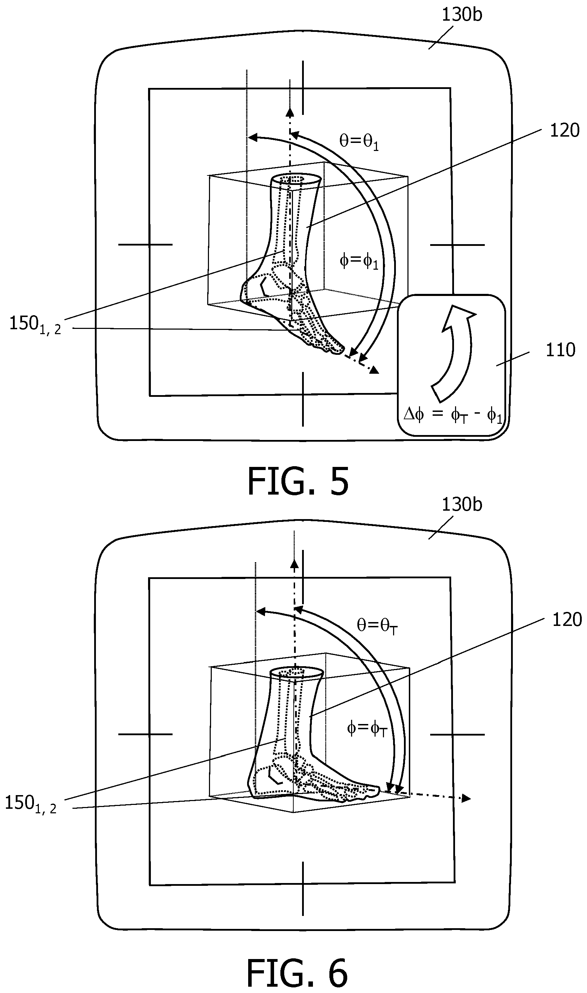

Fig. 4 is a schematic diagram illustrating a first example of an anatomical structure 120 in a target pose, α_{T}, with respect the projection X-ray imaging system 130b, in accordance with some aspects of the present disclosure. Fig. 4 has several features in common with Fig. 3. Features in Fig. 4 that share the same labels as Fig. 3 refer to the same feature, and a description of the feature is not duplicated for the sake of brevity. As compared to the anatomical structure 120 illustrated in Fig. 3, the pose of the anatomical structure 120 illustrated in Fig. 4 has been adjusted by tilting the Tibia so as to reduce the parameter α from the initial value α₁ to the target value α_{T}. Consequently, the anatomical structure 120 illustrated in Fig. 4 has the target pose with respect to the projection X-ray imaging system 130b. Fig. 5 is a schematic diagram illustrating a second example of an anatomical structure 120 in an initial pose, ϕ₁, with respect to a projection X-ray imaging system 130b, in accordance with some aspects of the present disclosure. In the example illustrated in Fig. 5, the pose is defined by the parameter ϕ. The parameter ϕ represents an angle of a line connecting the base of the Calcaneus, i.e. heel bone, and the first Metatarsal, with respect to a vertical orientation of the X-ray detector 130b.

Fig. 6 is a schematic diagram illustrating a second example of an anatomical structure 120 in a target pose, ϕ_{T}, with respect to a projection X-ray imaging system 130b, in accordance with some aspects of the present disclosure. Fig. 6 has several features in common with Fig. 5. Features in Fig. 6 that share the same labels as Fig. 5 refer to the same feature and a description of the feature is not duplicated for the sake of brevity. As compared to the anatomical structure 120 illustrated in Fig. 5, the pose of the anatomical structure 120 illustrated in Fig. 6 has been adjusted by reducing the parameter ϕ from the initial value ϕ₁ to the target value ϕ_{T}. Consequently, the anatomical structure 120 illustrated in Fig. 6 has the target pose with respect to the projection X-ray imaging system 130b.

A combination of the parameters α, and ϕ described above may also be used to define the pose of the anatomical structure 120 with respect to the projection X-ray imaging system. For instance, the parameters α, and ϕ, described above may both be used to define the pose of the anatomical structure 120 with respect to the projection X-ray imaging system. Together, the parameters α, and ϕ, also define a mutual positioning of anatomical features 150₁, 150₂, of the anatomical structure 120. In this case, α, and ϕ define a mutual positioning of the Tibia 150₁, and a line connecting the base of the Calcaneus, i.e. heel bone, and the first Metatarsal 150₂. Thus, as compared to the anatomical structure 120 illustrated in Fig. 5, the pose of the anatomical structure 120 illustrated in Fig. 6 represents an adjustment to a mutual positioning θ of the anatomical features 150₁ and 150₂. In this example, the angle θ, represents an amount of flexion between the anatomical features 150₁, and 150₂.

Other parameters may be used to define the initial pose of the anatomical structure 120 with respect to the projection X-ray imaging system. For instance, a rotation of the Tibia may be defined by a rotational angle that is measured around a vertical axis that is parallel to the surface of the X-ray detector 130b. A position of the medial process of the Talus may be defined by a vertical and a horizontal distance from the centre of the X-ray detector 130b. Such parameters may be used in addition to, or instead of, the example parameters α, and ϕ, described above, to define a pose of the ankle 120 with respect to the projection X-ray imaging system.

Returning to the flowchart illustrated in Fig. 7, in the operation S120, the image data, i.e. the initial X-ray projection image representing the anatomical structure 120 and/or the camera image representing the anatomical structure 120, are inputted into a neural network. In the operation S130, and in response to the inputting, the neural network generates pose adjustment information 110 for adjusting the initial pose α₁, ϕ₁ of the anatomical structure 120 with respect to the projection X-ray imaging system 130a, 130b in order to acquire the target X-ray projection image.

In general, the pose adjustment information 110 may be defined with respect to various datum. For instance, the pose adjustment information 110 may be defined with respect to the initial pose ϕ₁. Alternatively, the pose adjustment information 110 may be defined with respect to an orientation of a central axis 160 of the projection X-ray imaging system 130a, 130b. Alternatively, the pose adjustment information 110 may be defined with respect to an anatomy-specific coordinate system, e.g. the cranial-caudal axis, of the ventral-dorsal axis, or the medial-lateral axis, and so forth. Defining the pose adjustment information 110 with respect to the initial pose ϕ₁, or with respect to an anatomy-specific coordinate system facilitates ease of adjusting the pose of the anatomical structure 120, and also ease of annotating the images in the training data that is used to train the neural network.

With reference to the example illustrated in Fig. 3 and Fig. 4, in the operation S130, the neural network generates pose adjustment information 110 in the form of a change in the parameter α, i.e. Δα = α_{T} - α₁, for adjusting the initial pose α = α₁ of the anatomical structure 120 illustrated in Fig. 3, in order to obtain the target pose α = α_{T} with which to acquire the target X-ray projection image representing the anatomical structure illustrated in Fig. 4. Similarly, with reference to the example illustrated in Fig. 5 and Fig. 6, in this operation, the neural network generates pose adjustment information 110 in the form of a change in the parameter ϕ, i.e. Δϕ = ϕ_{T} - ϕ₁, for adjusting the initial pose ϕ = ϕ₁ of the anatomical structure 120 illustrated in Fig. 5, in order to obtain the target pose ϕ = ϕ_{T} with which to acquire the target X-ray projection image representing the anatomical structure illustrated in Fig. 6.

In one example, the anatomical structure 120 includes a plurality of anatomical features 150_{1, 2}, and the pose adjustment information 110 represents an adjustment to a mutual positioning θ of the anatomical features 150_{1, 2}. For instance, with reference to Fig. 5 and Fig. 6, the pose adjustment information may include an adjustment to the angle α, or the angle ϕ, and which consequently results in an adjustment to the angle θ, and which represents a mutual positioning of the anatomical features 150₁ and 150₂. Examples of other anatomical features, the positions of which may be adjusted in accordance with this example, in include bones, processes of bones, a shaft of a bone, gaps between bones, and so forth.

In another example, the pose adjustment information 110 comprises an adjustment to an absolute position and/or an absolute orientation of the anatomical structure 120. In this example, the anatomical structure is adjusted rather than the projection X-ray imaging system. The projection X-ray imaging system remains static, and consequently the desired image may be acquired in a more efficient manner, thereby improving workflow.

The neural network is trained to generate the pose adjustment information 110 using training data comprising a plurality of training images representing the anatomical structure 120, the training images respectively comprising X-ray projection images acquired with the anatomical structure in a current pose with respect the projection X-ray imaging system and/or camera images acquired by a camera configured to view the anatomical structure with the anatomical structure in the current pose with respect the projection X-ray imaging system, and wherein the training data further comprises, for each training image, corresponding pose adjustment information for adjusting the current pose of the anatomical structure with respect to the projection X-ray imaging system in order to acquire the target X-ray projection image.

The neural network may be provided by various types of architectures, including for example a convolutional neural network "CNN" architecture, or a transformer, or a recurrent neural network "RNN" architecture with unidirectional or bidirectional long short-term memory "LSTM" architecture, etc.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. Other loss functions like the Kullback-Leibler divergence may additionally be used when training a variational autoencoder to ensure that the distribution of latent space encodings generated from temporal sequences of training X-ray images is similar to a standard Gaussian distribution with mean 0 and standard deviation of 1. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

Thus, in general the neural network may be trained to generate the pose adjustment information 110 by:
- receiving the training data; and
- for each of a plurality of the training images in the training data:
   - inputting the training image into the neural network;
   - generating pose adjustment information using the neural network; and
   - adjusting parameters of the neural network based on a difference between the pose adjustment information generated by the neural network and the corresponding pose adjustment information from the training data; and
- repeating the inputting, and the generating, and the adjusting, until a stopping criterion is met.

The training data used in the training method described above may be acquired from historic imaging procedures. The training data may be acquired using an arrangement similar to that illustrated in Fig. 1 and Fig. 2, and wherein the projection X-ray imaging system 130a, 130b is used to acquire for each imaging procedure an X-ray projection image representing the anatomical structure and/or the camera 140, which is configured to view the anatomical structure 120, is used to acquire a camera image representing the anatomical structure 120. If the training images include camera images, a pose of the camera with respect to the projection X-ray imaging system may be the same for the camera used to acquire the camera images and the camera used to acquire the camera images at inference.

Each of the training images may be said to capture the anatomical structure with a current pose with respect to the projection X-ray imaging system. The training images are labelled, e.g. by an expert, with pose adjustment information 110 for adjusting the current pose of the anatomical structure with respect to the projection X-ray imaging system in order to acquire the target X-ray projection image. For instance, the training images may be labelled with pose adjustment information that includes adjustments Δα, and Δϕ to the parameters α, and ϕ, described above with reference to Fig. 2 - Fig. 6. The training images may be labelled with other pose adjustment information such as adjustments to a position of the anatomical structure, and so forth. Some tens, or hundreds, or thousands of training images from historic imaging procedures may be used to train the neural network. The training images may represent subjects having different ages, gender, body mass index, and so forth. The training images may include a variety of different pose adjustment information. Some of the training images may represent the anatomical structure in the target pose and are therefore labelled with pose adjustment information indicating that no pose adjustment is required in order to acquire the target X-ray projection image.

Returning to the flowchart illustrated in Fig. 7, in the operation S140, the pose adjustment information 110, is outputted. In general the pose adjustment information that is outputted 110 may include an identification of the pose parameter(s) to be adjusted and a magnitude of the adjustment that is required in order to obtain the target pose. The pose adjustment information 110 may be outputted in any human-comprehensible manner, including graphically, and audially, for example. The pose adjustment information 110 may also be outputted in various ways, such as using a projector, or on a display. For instance, the operation of outputting S140 the pose adjustment information 110 may include projecting a graphical representation of the pose adjustment information 110. A graphical representation of the pose adjustment information 110 may be outputted onto the anatomical structure, or onto a portion of the projection X-ray imaging system, or onto a wall, for example. The operation of outputting S140 the pose adjustment information 110 may alternatively include outputting the received initial X-ray projection image representing the anatomical structure 120, and outputting the pose adjustment information 110 as an overlay on the image. In this case, the image, and the overlay, may be outputted to a display such as a monitor. The operation of outputting S140 the pose adjustment information 110 may alternatively include outputting the received camera image representing the anatomical structure 120, and outputting the pose adjustment information 110 as an overlay on the image. In this case, the image, and the overlay, may again be outputted to a display such as a monitor.

Some examples of the operation of outputting S140 pose adjustment information 110, are illustrated in Fig. 3, and in Fig. 5. In these examples, a graphical representation of the pose adjustment information 110 is projected onto the X-ray detector 130b of the projection X-ray imaging system, and the pose adjustment information 110 includes a textual indication of the parameter to be adjusted, i.e. α and ϕ, a numerical indication of the magnitude of the adjustment, and an icon indicating the direction of the adjustment that is required in order to obtain the target pose α_{T}, and ϕ_{T}. More generally, the pose adjustment information 110 may be outputted in a numerical format or in a graphical format.

Subsequent to the operation S140, the pose adjustment information 110 may be used to adjust the pose of the anatomical structure 120, and the target X-ray projection image may be acquired.

Further variations of the above-described method are also contemplated, as described in the examples below.

In one example, the received image data comprises an X-ray projection image and/or a camera image acquired with the anatomical structure 120 in a target pose α_{T}, ϕ_{T} with respect the projection X-ray imaging system 130a, 130b. In this example, the method described with reference to the flowchart illustrated in Fig. 7 includes:
- recording the received image data for use as additional training data; and
- further training the neural network to generate the pose adjustment information 110 using the additional training data; and
- wherein the further training of the neural network is performed with the recorded image data and corresponding pose adjustment information indicating that no adjustment is to be made to the pose of the anatomical structure.

In this example, the training of the neural network is updated using additional training data that is acquired during inference. Consequently, over time, the neural network learns to generate the pose adjustment information from an increasingly large set of training data. This approach facilitates the deployment of the neural network following its training with a minimal set of training data, following which the accuracy of the pose adjustment information is improved over time due to the inclusion of the additional training data. Furthermore, the inherent variability within the newly-acquired training data facilitates the tailoring of the pose adjustment information to the preferences of the operator over time.

In this example, the recording of the received image data, may be performed based on user acceptance of the received image data as the additional training data. For instance, in this example, a prompt may be presented to a user via a display proposing the image for use as additional training data. If the operator considers the initial pose image suitable for acquiring a target X-ray projection image, the user may confirm the proposal, subsequent to which, further training of the neural network is performed. Groups of images may be recorded in this manner over time, subsequent to which the further training is performed with the group of images.

In another example, the training data that is used to train the neural network includes, for each training image, context information indicating a context of the training image. In this example, the neural network is trained to generate the pose adjustment information 110 based further on the context information, and the method described with reference to the flowchart illustrated in Fig. 7 includes:
- inputting the context information into the neural network; and
- generating the pose adjustment information 110 using the neural network based further on the context information.

An example of the context information that may be used in this example is a mobility constraint of the anatomical structure. For instance, a limb that is fixed in a cast, has limited mobility as compared to a healthy limb, and consequently it may not be possible to adjust the pose of the limb at-will. The mobility constraint may indicate that one or more anatomical features in the anatomical structure are not mutually mobile, for instance. The corresponding pose adjustment information for such training images takes into account the mobility constraint. For instance, the corresponding pose adjustment information may be limited to global adjustments of the pose of the anatomical structure that do not alter the mutual positioning of anatomical features within the anatomical structure. At inference time, the pose adjustment information 110 is generated by the neural network based on the mobility constraint, avoiding that un-obtainable poses of the anatomical structure are generated by the neural network.

Other examples of context information that may be used in this example include an indication of the rationale for acquiring the X-ray projection image, or an indication of a diagnosis of the subject, or an indication of subject data such as age, body mass index, and so forth. Such context information may also place constraints on the ability to adjust the pose of an anatomical structure at-will. For instance, a subject that has had a fall may have one set of constraints on their mobility, and a subject over a specified age may have another set of constraints on their mobility. Consequently, by generating the pose adjustment information 110 based on such context information, it may be avoided that un-obtainable poses of the anatomical structure are generated by the neural network.

In another example, a computer program product is provided. The computer program product comprises instructions which when executed by one or more processors 210, cause the one or more processors to carry out a method of providing pose adjustment information 110 for adjusting a pose of an anatomical structure 120 with respect to a projection X-ray imaging system 130a, 130b in order to acquire a target X-ray projection image representing the anatomical structure 120. The method comprises:
- receiving S110 image data, the image data comprising:
   - an initial X-ray projection image representing the anatomical structure 120, the initial X-ray projection image being acquired by the projection X-ray imaging system 130a, 130b with the anatomical structure 120 in an initial pose α₁, ϕ₁ with respect the projection X-ray imaging system 130a, 130b; and/or

   - a camera image representing the anatomical structure 120, the camera image being acquired by a camera 140 configured to view the anatomical structure 120, the camera image being acquired with the anatomical structure 120 in the initial pose α₁, ϕ₁ with respect the projection X-ray imaging system 130a, 130b;
- inputting S120 the image data into a neural network; and
- in response to the inputting, generating S130 using the neural network, pose adjustment information 110 for adjusting the initial pose α₁, ϕ₁ of the anatomical structure 120 with respect to the projection X-ray imaging system 130a, 130b in order to acquire the target X-ray projection image; and outputting S140 the pose adjustment information 110; and
- wherein the neural network is trained to generate the pose adjustment information 110 using training data comprising a plurality of training images representing the anatomical structure 120, the training images respectively comprising X-ray projection images acquired with the anatomical structure in a current pose with respect the projection X-ray imaging system and/or camera images acquired by a camera configured to view the anatomical structure with the anatomical structure in the current pose with respect the projection X-ray imaging system, and wherein the training data further comprises, for each training image, corresponding pose adjustment information for adjusting the current pose of the anatomical structure with respect to the projection X-ray imaging system in order to acquire the target X-ray projection image.

In another example, a system 100 for providing pose adjustment information 110 for adjusting a pose of an anatomical structure 120 with respect to a projection X-ray imaging system 130a, 130b in order to acquire a target X-ray projection image representing the anatomical structure 120, is provided. The system includes one or more processors 210 configured to:
- receive S110 image data, the image data comprising:
   - an initial X-ray projection image representing the anatomical structure 120, the initial X-ray projection image being acquired by the projection X-ray imaging system 130a, 130b with the anatomical structure 120 in an initial pose α₁, ϕ₁ with respect the projection X-ray imaging system 130a, 130b; and/or
   - a camera image representing the anatomical structure 120, the camera image being acquired by a camera 140 configured to view the anatomical structure 120, the camera image being acquired with the anatomical structure 120 in the initial pose α₁, ϕ₁ with respect the projection X-ray imaging system 130a, 130b;
- input S120 the image data into a neural network; and
- in response to the input, generate S130 using the neural network, pose adjustment information 110 for adjusting the initial pose α₁, ϕ₁ of the anatomical structure 120 with respect to the projection X-ray imaging system 130a, 130b in order to acquire the target X-ray projection image; and
- output S140 the pose adjustment information 110; and
- wherein the neural network is trained to generate the pose adjustment information 110 using training data comprising a plurality of training images representing the anatomical structure 120, the training images respectively comprising X-ray projection images acquired with the anatomical structure in a current pose with respect the projection X-ray imaging system and/or camera images acquired by a camera configured to view the anatomical structure with the anatomical structure in the current pose with respect the projection X-ray imaging system, and wherein the training data further comprises, for each training image, corresponding pose adjustment information for adjusting the current pose of the anatomical structure with respect to the projection X-ray imaging system in order to acquire the target X-ray projection image.

An example of the system 100 is illustrated in Fig. 1 and in Fig. 2. It is noted that the system 100 may also include one or more of: a projection X-ray imaging system 130a, 130b for acquiring X-ray projection images; a camera 140 for acquiring camera images; a display (not illustrated in Fig. 1 for displaying the acquired images, the pose adjustment information 110, and so forth; and a user input device (not illustrated in Fig. 1 configured to receive user input fur use in relation to the method described above, such as a keyboard, a mouse, a touchscreen, and so forth.

In another aspect of the present disclosure, the one or more processors 210 of the system 100 described above with reference to Fig. 1 and in Fig. 2 are configured to perform another method of providing pose adjustment information. In this aspect, a computer-implemented method of providing pose adjustment information 310 for adjusting a pose α₂, ϕ₂ of an anatomical structure 120 with respect to a projection X-ray imaging system 130a, 130b in order to acquire a target X-ray projection image 310_{T} representing the anatomical structure 120, is provided. The method includes:
- receiving S310 image data, the image data comprising:
   - an initial X-ray projection image 310₁ representing the anatomical structure 120, the initial X-ray projection image being acquired by the projection X-ray imaging system 130a, 130b with the anatomical structure 120 in an initial pose α₁, ϕ₁ with respect the projection X-ray imaging system 130a, 130b;
   - an initial camera image 320₁ representing the anatomical structure 120, the initial camera image being acquired by a camera 140 configured to view the anatomical structure 120, the initial camera image being acquired with the anatomical structure 120 in the initial pose α₁, ϕ₁ with respect the projection X-ray imaging system 130a, 130b; and
   - a subsequent camera image 320₂ representing the anatomical structure 120, the subsequent camera image being acquired by a camera 140 configured to view the anatomical structure 120, the subsequent camera image 320₂ being acquired with the anatomical structure 120 in a subsequent pose α₂, ϕ₂ with respect the projection X-ray imaging system 130a, 130b and at a later point in time T₂ to the initial camera image 320₁;
- determining S320, based on the initial X-ray projection image 310₁, pose adjustment information 110 for adjusting the initial pose α₁, ϕ₁ of the anatomical structure 120 with respect to the projection X-ray imaging system 130a, 130b in order to acquire the target X-ray projection image 310_{T} representing the anatomical structure 120;
- determining S330 a registration 330 between the anatomical structure 120 in the initial X-ray projection image 310₁ and the anatomical structure 120 in the initial camera image 320₁;
- deforming S340 the initial camera image 320₁ using the registration 330 and the pose adjustment information 110 to provide a target camera image 320_{T} corresponding to the target X-ray projection image 310_{T};
- determining S350 the pose adjustment information 310 for adjusting the subsequent pose α₂, ϕ₂ of the anatomical structure 120 with respect to the projection X-ray imaging system 130a, 130b in order to acquire the target X-ray projection image 310_{T} representing the anatomical structure 120, based on a deviation between the subsequent camera image 320₂ and the target camera image 320_{T}; and
- outputting S360 the pose adjustment information 310.

This method provides pose adjustment information that may be used to adjust an initial pose of an anatomical structure with respect to the projection X-ray imaging system in order to acquire a target X-ray projection image representing the anatomical structure, or in other words, a desired image. An initial X-ray projection image, and an initial camera image, are acquired with the anatomical structure in the initial pose. The initial X-ray projection image may deviate from the target X-ray projection image. Pose adjustment information is then generated to determine how the anatomical structure in the initial X-ray projection image should be adjusted in order to acquire the target X-ray projection image. This operation may be performed using a neural network, as described in the aforementioned aspect. The registration operation and the deformation operation, are then used in combination with the pose adjustment information to provide a target camera image, i.e. an image showing how the anatomical structure should have appeared if the target X-ray projection image had been acquired. At a later point in time, a subsequent camera image is acquired with the anatomical structure in a subsequent pose. A deviation between the subsequent camera image and the target camera image is used to provide pose adjustment information for acquiring the target X-ray projection image. Since, in this method, the pose adjustment information is generated using the deviation between the subsequent camera image and the target camera image, the target X-ray projection image is acquired by positioning the anatomical structure without the need to acquire a subsequent X-ray projection image. This has the advantage of providing the target X-ray projection image with reduced X-ray dose to the subject.

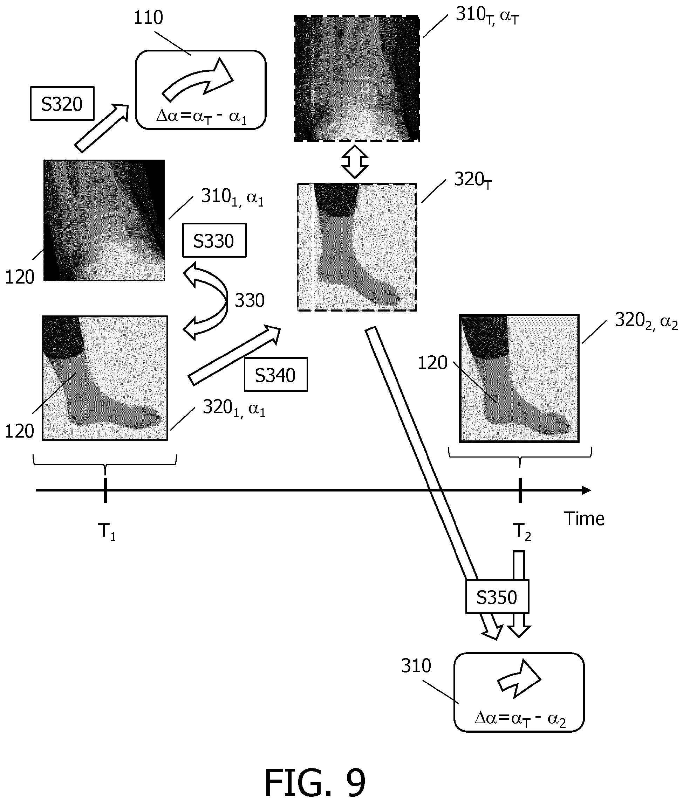

Examples in accordance with this aspect are described with reference to Fig. 8, and Fig. 9. Fig. 8 is a flowchart illustrating an example of a method of providing pose adjustment information for adjusting a pose of an anatomical structure with respect to a projection X-ray imaging system in order to acquire a target X-ray projection image representing the anatomical structure, in accordance with some aspects of the present disclosure.

With reference to the above method and the flowchart illustrated in Fig. 8, in the operation S310, image data is received. The image data that is received in the operation S110 includes an initial X-ray projection image 310₁ representing the anatomical structure 120, an initial camera image 320₁ representing the anatomical structure 120, and a subsequent camera image 320₂ representing the anatomical structure 120. Examples of these images are illustrated in Fig. 9, which is a schematic diagram illustrating an example of a method of providing pose adjustment information 310 for adjusting a pose α₂ of an anatomical structure 120 with respect to a projection X-ray imaging system in order to acquire a target X-ray projection image 310_{T} representing the anatomical structure 120, in accordance with some aspects of the present disclosure.

The initial X-ray projection image 310₁ is acquired by the projection X-ray imaging system 130a, 130b with the anatomical structure 120 in an initial pose α₁, ϕ₁ with respect the projection X-ray imaging system 130a, 130b. The initial camera image 320₁ is acquired by a camera 140 configured to view the anatomical structure 120. The initial camera image is acquired with the anatomical structure 120 in the initial pose α₁, ϕ₁ with respect the projection X-ray imaging system 130a, 130b. The initial X-ray projection image 310₁ and the initial camera image 320₁ may be acquired concurrently, and at a first point in time, T₁, as illustrated in the timeline in Fig. 9. The subsequent camera image 320₂ is acquired by a camera 140 configured to view the anatomical structure 120. The subsequent camera image 320₂ is acquired with the anatomical structure 120 in a subsequent pose α₂, ϕ₂ with respect the projection X-ray imaging system 130a, 130b and at a later point in time T₂ to the initial camera image 320₁. An example of the subsequent camera image 320₂ is illustrated on the right-hand side of Fig. 9 and at the time T₂ on the timeline. In general, the camera images may be optical camera images acquired by an optical camera and/or depth camera images acquired by a depth camera. The camera 140 may also view at least a portion of the projection X-ray imaging system. For instance, the camera 140 may also view at least a portion of the X-ray source 130a, or at least a portion of the X-ray detector 130b. Thus, the camera 140 may acquire camera images representing the anatomical structure and at least a portion of the projection X-ray imaging system 130a, 130b.

The initial X-ray projection image 310₁, and also the initial camera image 320₁, are acquired with the anatomical structure 120 in an initial pose α₁, ϕ₁ with respect the projection X-ray imaging system 130a, 130b. Examples of the initial pose are described above with reference to Fig. 3 and Fig. 5. The subsequent camera image 320₂ is acquired at a later point in time, T₂. There may be a time delay of some seconds, minutes, hours, days, or longer between the first point in time T₁ at which the anatomical structure 120 in the initial pose α₁, ϕ₁, and the second point in time at which the anatomical structure 120 is in the subsequent pose α₂, ϕ₂. Consequently, the subsequent pose α₂, ϕ₂ of the anatomical structure 120 in the subsequent camera image 320₂ may differ from the initial pose α₁, ϕ₁. This is illustrated by way of the different poses in the camera images 320₁ and 320₂.

The initial X-ray projection image 310₁ may be received from the projection X-ray imaging system 130a, 130b illustrated in Fig. 1, and the initial and subsequent camera images 320₁ and 320₂ may be received from the camera 140 illustrated in Fig. 1. In general, the image data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

Returning to the flowchart illustrated in Fig. 8, in the operation S320, pose adjustment information 110 is determined for adjusting the initial pose α₁, ϕ₁ of the anatomical structure 120 with respect to the projection X-ray imaging system 130a, 130b in order to acquire the target X-ray projection image 310_{T} representing the anatomical structure 120. This operation is illustrated in the top-left portion of Fig. 9. The pose adjustment information 110 is determined based on the initial X-ray projection image 310₁. The pose adjustment information determines how the anatomical structure in the initial X-ray projection image should be adjusted in order to acquire the target X-ray projection image 310_{T}.

The operation S320 may be performed using a neural network, as described in the aforementioned aspect. In other words, the operation S320 may be performed using the neural network described above with reference to the operations S120, S130, and S140. Thus, the operation of determining S320 pose adjustment information 110 for adjusting the initial pose α₁, ϕ₁ of the anatomical structure 120 with respect to the projection X-ray imaging system 130a, 130b, may include:
- inputting the initial X-ray projection image 310₁ into a neural network; and
- wherein the neural network is trained to generate the pose adjustment information 110 using training data comprising a plurality of training images representing the anatomical structure 120, the training images comprising X-ray projection images acquired with the anatomical structure 120 in a current pose with respect the projection X-ray imaging system 130a, 130b, and wherein the training data further comprises, for each training image, corresponding pose adjustment information for adjusting the current pose of the anatomical structure with respect to the projection X-ray imaging system 130a, 130b in order to acquire the target X-ray projection image 310_{T} representing the anatomical structure 120.

The operation S320 may be performed based further on the initial camera image 320₁ representing the anatomical structure 120, as also described above in the operations S120, S130, and S140. Alternatively, the pose adjustment information may be determined in accordance with a technique disclosed in a document by Krönke, S., et al., "CNN-based pose estimation for assessing quality of ankle-joint X-ray images," Proc. SPIE 12032, Medical Imaging 2022: Image Processing, 120321A, 4 April 2022.

Returning to the flowchart illustrated in Fig. 8, in the operation S330, a registration 330 between the anatomical structure 120 in the initial X-ray projection image 310₁ and the anatomical structure 120 in the initial camera image 320₁ is determined. The registration operation S330 provides a mapping between the features in the initial X-ray projection image 310₁ and the corresponding features in the initial camera image 320₁. The registration operation S330 may be performed by fitting a template kinematic model representing the anatomical structure 120 to the initial X-ray projection image 310₁ and the initial camera image 320₁. For instance, a template kinematic model representing bone and tissue may be fitted to the initial X-ray projection image 310₁ by scaling the dimensions of features in the kinematic model and adjusting their poses. The registration 330 may a deformable registration, or an affine registration, for example. Thus, in one example, the anatomical structure 120 comprises one or more bones and tissue surrounding the one or more bones, and the operation S330 of determining a registration 330 between the anatomical structure 120 in the initial X-ray projection image 310₁ and the anatomical structure 120 in the initial camera image 320₁, is performed using a kinematic model representing the bones and the tissue.

With continued reference to the flowchart illustrated in Fig. 8, in the operation S340, the initial camera image 320₁ is deformed using the registration 330 and the pose adjustment information 110 to provide a target camera image 320_{T} corresponding to the target X-ray projection image 310_{T}. The resulting target camera image 320_{T} therefore indicates how the anatomical structure should appear in order to acquire the target X-ray projection image 310_{T}. The deforming operation S340 may be performed using known image processing techniques. For instance, the deforming operation may include performing a rigid, or a non-rigid, or an elastic deformation of the initial camera image 320₁. This results in a target camera image 320_{T} corresponding to the target X-ray projection image 310_{T}, as illustrated in the central portion of Fig. 9.

Returning to the flowchart illustrated in Fig. 8, in the operation S350, pose adjustment information 310 is determined, based on a deviation between the subsequent camera image 320₂ and the target camera image 320₁. The pose adjustment information 310 is suitable for adjusting the subsequent pose α₂, ϕ₂ of the anatomical structure 120 with respect to the projection X-ray imaging system 130a, 130b in order to acquire the target X-ray projection image 310_{T} representing the anatomical structure 120. This operation is illustrated in the lower right-hand portion of Fig. 9. In the operation S350, the pose adjustment information 310 is determined based on a deviation between the subsequent camera image 320₂ and the target camera image 320₁. The operation S350 may be performed using various techniques, including using a trained model, or a statistical model. For example, a trained model may be trained in a similar manner to the neural network described above with reference to the operations S120, S130, and S140. Thus, the trained model may be trained to generate the pose adjustment information using training data that includes training camera images representing the anatomical structure, and corresponding pose adjustment information that is provided by expert annotations. Alternatively, the operation S350 may be performed using a statistical model.

The pose adjustment information 310 that is provided by the operation S350 facilitates adjustments to be made to the subsequent pose α₂, ϕ₂ without the need to acquire an X-ray projection image with the anatomical structure in the subsequent pose α₂, ϕ₂. Consequently, the anatomical structure may be adjusted to provide a pose that is suitable for acquiring the target X-ray projection image 310_{T} with reduced X-ray dose to the subject.

Returning to the flowchart illustrated in Fig. 8, in the operation S360 the pose adjustment information 310 is outputted. The pose adjustment information 310 may be outputted in a similar manner to that described above with reference to Fig. 3, and Fig. 5. Thus, the pose adjustment information 110 may be outputted in any human-comprehensible manner, including graphically, and audially, for example.

By way of some examples, the operation of outputting S360 the pose adjustment information 310 may include:
- projecting a graphical representation of the pose adjustment information 310; or
- outputting the received subsequent camera image 320₂ representing the anatomical structure 120; and
- outputting the pose adjustment information 310 as an overlay on the received subsequent camera image 320₂.

In some examples, the pose adjustment information 110 may be outputted in a numerical format or in a graphical format.

Subsequent to the operation S360, the pose adjustment information 310 may be used to adjust the pose of the anatomical structure 120, and the target X-ray projection image 310_{T} may be acquired.

Further variations of the above-described method are also contemplated, as described in the examples below.

In one example, the operation of receiving a subsequent camera image 320₂ representing the anatomical structure 120, is performed iteratively. In this example, the following operations are performed in each iteration:
- determining S350 the pose adjustment information 310 for adjusting the subsequent pose α₂, ϕ₂ of the anatomical structure 120 with respect to the projection X-ray imaging system 130a, 130b in order to acquire the target X-ray projection image 310_{T} representing the anatomical structure 120, based on a deviation between the subsequent camera image 320₂ and the target camera image 320_{T}; and
- outputting S360 the pose adjustment information 310.

The iterative pose adjustment information that is provided in this example facilitates the repeated re-positioning of the anatomical structure until a target pose α_{T}, ϕ_{T} is reached for acquiring the target X-ray projection image 310_{T}. The iterations may be performed substantially in real-time. This facilitates the live re-positioning of the anatomical structure and facilitates adjustments to the pose to be made that compensate for patient motion in the time interval between the acquisition of the subsequent camera image 320₂ and the acquisition of the target X-ray projection image 310_{T}.

In another example, the pose adjustment information 310 for adjusting the initial pose α₁, ϕ₁ of the anatomical structure 120 with respect to the projection X-ray imaging system 130a, 130b in order to acquire the target X-ray projection image 310_{T} representing the anatomical structure 120, comprises an adjustment to an absolute position and/or an absolute orientation of the anatomical structure 120. In this example, the anatomical structure is adjusted rather than the projection X-ray imaging system. The projection X-ray imaging system remains static, and consequently the desired image may be acquired in a more efficient manner, thereby improving workflow.

In another example, the anatomical structure 120 includes a plurality of anatomical features 150₁, 150₂, and the pose adjustment information 310 for adjusting the initial pose α₁, ϕ₁ of the anatomical structure 120 with respect to the projection X-ray imaging system 130a, 130b represents an adjustment to a mutual positioning ϕ of the anatomical features. Examples of other anatomical features, the positions of which may be adjusted in accordance with this example, in include bones, processes of bones, a shaft of a bone, gaps between bones, and so forth.

In another example, the pose adjustment information 310 is defined with respect to the initial pose α₁, ϕ₁, or wherein the pose adjustment information 310 is defined with respect to an orientation of a central axis 160 of the projection X-ray imaging system 130a, 130b. Alternatively, the pose adjustment information 110 may be defined with respect to an anatomy-specific coordinate system, e.g. the cranial-caudal axis, of the ventral-dorsal axis, or the medial-lateral axis, and so forth. Defining the pose adjustment information 110 with respect to the initial pose ϕ₁, or with respect to an anatomy-specific coordinate system facilitates ease of annotating the images in the training data.

In another example, the initial X-ray projection image 310₁ comprises a relatively lower X-ray dose than the target X-ray projection image 310_{T}. Such an initial X-ray projection image 310₁ may be referred-to as a "scout scan". This example has the advantage of reducing the total amount of X-ray dose to the subject.

In another example, a computer program product is provided. The computer program product comprises instructions which when executed by one or more processors 210, cause the one or more processors 210 to carry out a method of providing pose adjustment information 310 for adjusting a pose α₂, ϕ₂ of an anatomical structure 120 with respect to a projection X-ray imaging system 130a, 130b in order to acquire a target X-ray projection image 310_{T} representing the anatomical structure 120, the method comprising:
- receiving S310 image data, the image data comprising:
   - an initial X-ray projection image 310₁ representing the anatomical structure 120, the initial X-ray projection image being acquired by the projection X-ray imaging system 130a, 130b with the anatomical structure 120 in an initial pose α₁, ϕ₁ with respect the projection X-ray imaging system 130a, 130b;
   - an initial camera image 320₁ representing the anatomical structure 120, the initial camera image being acquired by a camera 140 configured to view the anatomical structure 120, the initial camera image being acquired with the anatomical structure 120 in the initial pose α₁, ϕ₁ with respect the projection X-ray imaging system 130a, 130b; and
   - a subsequent camera image 320₂ representing the anatomical structure 120, the subsequent camera image being acquired by a camera 140 configured to view the anatomical structure 120, the subsequent camera image 320₂ being acquired with the anatomical structure 120 in a subsequent pose α₂, ϕ₂ with respect the projection X-ray imaging system 130a, 130b and at a later point in time T₂ to the initial camera image 320₁;
- determining S320, based on the initial X-ray projection image 310₁, pose adjustment information 110 for adjusting the initial pose α₁, ϕ₁ of the anatomical structure 120 with respect to the projection X-ray imaging system 130a, 130b in order to acquire the target X-ray projection image 310_{T} representing the anatomical structure 120;
- determining S330 a registration 330 between the anatomical structure 120 in the initial X-ray projection image 310₁ and the anatomical structure 120 in the initial camera image 320₁;
- deforming S340 the initial camera image 320₁ using the registration 330 and the pose adjustment information 110 to provide a target camera image 320_{T} corresponding to the target X-ray projection image 310_{T};
- determining S350 the pose adjustment information 310 for adjusting the subsequent pose α₂, ϕ₂ of the anatomical structure 120 with respect to the projection X-ray imaging system 130a, 130b in order to acquire the target X-ray projection image 310_{T} representing the anatomical structure 120, based on a deviation between the subsequent camera image 320₂ and the target camera image 320_{T}; and
- outputting S360 the pose adjustment information 310.

In another example, a system 100 for providing pose adjustment information 310 for adjusting a pose α₂, ϕ₂ of an anatomical structure 120 with respect to a projection X-ray imaging system 130a, 130b in order to acquire a target X-ray projection image 310_{T} representing the anatomical structure 120, is provided. The system 100 comprises one or more processors 210 configured to:
- receive S310 image data, the image data comprising:
   - an initial X-ray projection image 310₁ representing the anatomical structure 120, the initial X-ray projection image being acquired by the projection X-ray imaging system 130a, 130b with the anatomical structure 120 in an initial pose α₁, ϕ₁ with respect the projection X-ray imaging system 130a, 130b;

   - an initial camera image 320₁ representing the anatomical structure 120, the initial camera image being acquired by a camera 140 configured to view the anatomical structure 120, the initial camera image being acquired with the anatomical structure 120 in the initial pose α₁, ϕ₁ with respect the projection X-ray imaging system 130a, 130b; and
   - a subsequent camera image 320₂ representing the anatomical structure 120, the subsequent camera image being acquired by a camera 140 configured to view the anatomical structure 120, the subsequent camera image 320₂ being acquired with the anatomical structure 120 in a subsequent pose α₂, ϕ₂ with respect the projection X-ray imaging system 130a, 130b and at a later point in time T₂ to the initial camera image 320₁;
   - determine S320, based on the initial X-ray projection image 310₁, pose adjustment information 110 for adjusting the initial pose α₁, ϕ₁ of the anatomical structure 120 with respect to the projection X-ray imaging system 130a, 130b in order to acquire the target X-ray projection image 310_{T} representing the anatomical structure 120;
   - determine S330 a registration 330 between the anatomical structure 120 in the initial X-ray projection image 310₁ and the anatomical structure 120 in the initial camera image 320₁;
   - deform S340 the initial camera image 320₁ using the registration 330 and the pose adjustment information 110 to provide a target camera image 320_{T} corresponding to the target X-ray projection image 310_{T};
   - determine S350 the pose adjustment information 310 for adjusting the subsequent pose α₂, ϕ₂ of the anatomical structure 120 with respect to the projection X-ray imaging system 130a, 130b in order to acquire the target X-ray projection image 310_{T} representing the anatomical structure 120, based on a deviation between the subsequent camera image 320₂ and the target camera image 320₁; and
   - output S360 the pose adjustment information 310.

An example of the system 100 is illustrated in Fig. 1 and in Fig. 2. It is noted that the system 100 may also include one or more of: a projection X-ray imaging system 130a, 130b for acquiring X-ray projection images; a camera 140 for acquiring camera images; a display (not illustrated in Fig. 1 for displaying the acquired images, the pose adjustment information 110, and so forth; and a user input device (not illustrated in Fig. 1 configured to receive user input fur use in relation to the method described above, such as a keyboard, a mouse, a touchscreen, and so forth.

The above examples are to be understood as illustrative of the present disclosure, and not restrictive. Further examples are also contemplated. For instance, the examples described in relation to a system may also be provided by the corresponding computer-implemented method, or by the corresponding computer program product, or by the corresponding computer-readable storage medium. It is to be understood that a feature described in relation to any one example may be used alone, or in combination with other described features, and may be used in combination with one or more features of another of the examples, or a combination of other examples. Furthermore, modifications not described above may also be employed without departing from the scope of the invention, which is defined in the accompanying claims. **In** the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of providing pose adjustment information (310) for adjusting a pose (α₂, ϕ₂) of an anatomical structure (120) with respect to a projection X-ray imaging system ((130a, 130b)) in order to acquire a target X-ray projection image (310_{T}) representing the anatomical structure (120), the method comprising:
receiving (S310) image data, the image data comprising:
an initial X-ray projection image (310₁) representing the anatomical structure (120), the initial X-ray projection image being acquired by the projection X-ray imaging system (130a, 130b) with the anatomical structure (120) in an initial pose (α₁, ϕ₁) with respect the projection X-ray imaging system (130a, 130b);
an initial camera image (320₁) representing the anatomical structure (120), the initial camera image being acquired by a camera (140) configured to view the anatomical structure (120), the initial camera image being acquired with the anatomical structure (120) in the initial pose (α₁, ϕ₁) with respect the projection X-ray imaging system (130a, 130b); and
a subsequent camera image (320₂) representing the anatomical structure (120), the subsequent camera image being acquired by a camera (140) configured to view the anatomical structure (120), the subsequent camera image (320₂) being acquired with the anatomical structure (120) in a subsequent pose (α₂, ϕ₂) with respect the projection X-ray imaging system (130a, 130b) and at a later point in time (T₂) to the initial camera image (320₁);
determining (S320), based on the initial X-ray projection image (310₁) pose adjustment information (110) for adjusting the initial pose (α₁, ϕ₁) of the anatomical structure (120) with respect to the projection X-ray imaging system (130a, 130b) in order to acquire the target X-ray projection image (310_{T}) representing the anatomical structure (120);
determining (S330) a registration (330) between the anatomical structure (120) in the initial X-ray projection image (310₁) and the anatomical structure (120) in the initial camera image (320₁);
deforming (S340) the initial camera image (320₁) using the registration (330) and the pose adjustment information (110) to provide a target camera image (320₁) corresponding to the target X-ray projection image (310_{T});
determining (S350) the pose adjustment information (310) for adjusting the subsequent pose (α₂, ϕ₂) of the anatomical structure (120) with respect to the projection X-ray imaging system (130a, 130b) in order to acquire the target X-ray projection image (310_{T}) representing the anatomical structure (120), based on a deviation between the subsequent camera image (320₂) and the target camera image (320_{T}); and
outputting (S360) the pose adjustment information (310).

2. The computer-implemented method according to Claim 1, wherein the camera images comprise optical camera images acquired by an optical camera and/or depth camera images acquired by a depth camera.

3. The computer-implemented method according to Claim 1, wherein the camera (140) is further configured to view at least a portion of the projection X-ray imaging system (130a, 130b), and wherein the camera image (140) further represents the at least a portion of the projection X-ray imaging system (130a, 130b).

4. The computer-implemented method according to Claim 1, wherein the determining (S320) pose adjustment information (110) for adjusting the initial pose (α₁, ϕ₁) of the anatomical structure (120) with respect to the projection X-ray imaging system (130a, 130b), comprises:
inputting the initial X-ray projection image (310₁) into a neural network; and
wherein the neural network is trained to generate the pose adjustment information (110) using training data comprising a plurality of training images representing the anatomical structure (120), the training images comprising X-ray projection images acquired with the anatomical structure (120) in a current pose with respect the projection X-ray imaging system (130a, 130b), and wherein the training data further comprises, for each training image, corresponding pose adjustment information for adjusting the current pose of the anatomical structure with respect to the projection X-ray imaging system (130a, 130b) in order to acquire the target X-ray projection image (310_{T}) representing the anatomical structure (120).

5. The computer-implemented method according to any previous Claim, wherein the anatomical structure (120) comprises one or more bones and tissue surrounding the one or more bones, and wherein the determining (S330) a registration (330) between the anatomical structure (120) in the initial X-ray projection image (310₁) and the anatomical structure (120) in the initial camera image (320₁), is performed using a kinematic model representing the bones and the tissue.

6. The computer-implemented method according to any previous Claim, wherein the operation of receiving a subsequent camera image (320₂) representing the anatomical structure (120), is performed iteratively; and wherein the following operations are performed in each iteration:
determining (S350) the pose adjustment information (310) for adjusting the subsequent pose (α₂, ϕ₂) of the anatomical structure (120) with respect to the projection X-ray imaging system (130a, 130b) in order to acquire the target X-ray projection image (310_{T}) representing the anatomical structure (120), based on a deviation between the subsequent camera image (320₂) and the target camera image (320_{T}); and
outputting (S360) the pose adjustment information (310).

7. The computer-implemented method according to Claim 1, wherein the pose adjustment information (310) for adjusting the initial pose (α₁, ϕ₁) of the anatomical structure (120) with respect to the projection X-ray imaging system (130a, 130b) in order to acquire the target X-ray projection image (310_{T}) representing the anatomical structure (120), comprises an adjustment to an absolute position and/or an absolute orientation of the anatomical structure (120).

8. The computer-implemented method according to Claim 1, wherein the anatomical structure (120) includes a plurality of anatomical features (150₁, 150₂), and wherein the pose adjustment information (310) for adjusting the initial pose (α₁, ϕ₁) of the anatomical structure (120) with respect to the projection X-ray imaging system (130a, 130b) represents an adjustment to a mutual positioning (ϕ) of the anatomical features.

9. The computer-implemented method according to Claim 1, wherein the pose adjustment information (310) is defined with respect to the initial pose (α₁, ϕ₁), or wherein the pose adjustment information (310) is defined with respect to an orientation of a central axis (160) of the projection X-ray imaging system (130a, 130b).

10. The computer-implemented method according to any previous Claim, wherein the outputting (S360) the pose adjustment information (310) comprises:
projecting a graphical representation of the pose adjustment information (310); or
outputting the received subsequent camera image (320₂) representing the anatomical structure (120); and
outputting the pose adjustment information (310) as an overlay on the received subsequent camera image (320₂).

11. The computer-implemented method according to Claim 10, wherein the outputting (S360) the pose adjustment information (310) as an overlay on the subsequent camera image (320₂), comprises outputting the pose adjustment information (310) in a numerical format, or outputting the pose adjustment information in a graphical format (310).

12. A computer program product comprising instructions which when executed by one or more processors (210), cause the one or more processors (210) to carry out a method of providing pose adjustment information (310) for adjusting a pose (α₂, ϕ₂) of an anatomical structure (120) with respect to a projection X-ray imaging system (130a, 130b) in order to acquire a target X-ray projection image (310_{T}) representing the anatomical structure (120), the method comprising:
receiving (S310) image data, the image data comprising:
an initial X-ray projection image (310₁) representing the anatomical structure (120), the initial X-ray projection image being acquired by the projection X-ray imaging system (130a, 130b) with the anatomical structure (120) in an initial pose (α₁, ϕ₁) with respect the projection X-ray imaging system (130a, 130b);
an initial camera image (320₁) representing the anatomical structure (120), the initial camera image being acquired by a camera (140) configured to view the anatomical structure (120), the initial camera image being acquired with the anatomical structure (120) in the initial pose (α₁, ϕ₁) with respect the projection X-ray imaging system (130a, 130b); and
a subsequent camera image (320₂) representing the anatomical structure (120), the subsequent camera image being acquired by a camera (140) configured to view the anatomical structure (120), the subsequent camera image (320₂) being acquired with the anatomical structure (120) in a subsequent pose (α₂, ϕ₂) with respect the projection X-ray imaging system (130a, 130b) and at a later point in time (T₂) to the initial camera image (320₁);
determining (S320), based on the initial X-ray projection image (310₁), pose adjustment information (110) for adjusting the initial pose (α₁, ϕ₁) of the anatomical structure (120) with respect to the projection X-ray imaging system (130a, 130b) in order to acquire the target X-ray projection image (310_{T}) representing the anatomical structure (120);
determining (S330) a registration (330) between the anatomical structure (120) in the initial X-ray projection image (310₁) and the anatomical structure (120) in the initial camera image (320₁);
deforming (S340) the initial camera image (320₁) using the registration (330) and the pose adjustment information (110) to provide a target camera image (320₁) corresponding to the target X-ray projection image (310_{T});
determining (S350) the pose adjustment information (310) for adjusting the subsequent pose (α₂, ϕ₂) of the anatomical structure (120) with respect to the projection X-ray imaging system (130a, 130b) in order to acquire the target X-ray projection image (310_{T}) representing the anatomical structure (120), based on a deviation between the subsequent camera image (320₂) and the target camera image (320_{T}); and
outputting (S360) the pose adjustment information (310).

13. A system (100) for providing pose adjustment information (310) for adjusting a pose (α₂, ϕ₂) of an anatomical structure (120) with respect to a projection X-ray imaging system (130a, 130b) in order to acquire a target X-ray projection image (310_{T}) representing the anatomical structure (120), the system (100) comprising one or more processors (210) configured to:
receive (S310) image data, the image data comprising:
an initial X-ray projection image (310₁) representing the anatomical structure (120), the initial X-ray projection image being acquired by the projection X-ray imaging system (130a, 130b) with the anatomical structure (120) in an initial pose (α₁, ϕ₁) with respect the projection X-ray imaging system (130a, 130b);
an initial camera image (320₁) representing the anatomical structure (120), the initial camera image being acquired by a camera (140) configured to view the anatomical structure (120), the initial camera image being acquired with the anatomical structure (120) in the initial pose (α₁, ϕ₁) with respect the projection X-ray imaging system (130a, 130b); and
a subsequent camera image (320₂) representing the anatomical structure (120), the subsequent camera image being acquired by a camera (140) configured to view the anatomical structure (120), the subsequent camera image (320₂) being acquired with the anatomical structure (120) in a subsequent pose (α₂, ϕ₂) with respect the projection X-ray imaging system (130a, 130b) and at a later point in time (T₂) to the initial camera image (320₁);
determine (S320), based on the initial X-ray projection image (310₁), pose adjustment information (110) for adjusting the initial pose (α₁, ϕ₁) of the anatomical structure (120) with respect to the projection X-ray imaging system (130a, 130b) in order to acquire the target X-ray projection image (310_{T}) representing the anatomical structure (120);
determine (S330) a registration (330) between the anatomical structure (120) in the initial X-ray projection image (310₁) and the anatomical structure (120) in the initial camera image (320₁);
deform (S340) the initial camera image (320₁) using the registration (330) and the pose adjustment information (110) to provide a target camera image (320₁) corresponding to the target X-ray projection image (310_{T});
determine (S350) the pose adjustment information (310) for adjusting the subsequent pose (α₂, ϕ₂) of the anatomical structure (120) with respect to the projection X-ray imaging system (130a, 130b) in order to acquire the target X-ray projection image (310_{T}) representing the anatomical structure (120), based on a deviation between the subsequent camera image (320₂) and the target camera image (320_{T}); and
output (S360) the pose adjustment information (310).

## Patentansprüche

1. Computerimplementiertes Verfahren zum Bereitstellen von Lageanpassungsinformationen (310) zum Anpassen einer Lage (α₂, ϕ₂) einer anatomischen Struktur (120) in Bezug auf ein Projektionsröntgenbildgebungssystem ((130a, 130b)), um ein Zielröntgenprojektionsbild (310_{T}) aufzunehmen, das die anatomische Struktur (120) darstellt, wobei das Verfahren Folgendes umfasst:
Empfangen (S310) von Bilddaten, wobei die Bilddaten Folgendes umfassen:
ein anfängliches Röntgenprojektionsbild (310₁), das die anatomische Struktur (120) darstellt, wobei das anfängliche Röntgenprojektionsbild vom Projektionsröntgenbildgebungssystem (130a, 130b) mit der anatomischen Struktur (120) in einer anfänglichen Lage (α₁, ϕ₁) in Bezug auf das Projektionsröntgenbildgebungssystem (130a, 130b) aufgenommen wird;
ein anfängliches Kamerabild (320₁), das die anatomische Struktur (120) darstellt, wobei das anfängliche Kamerabild von einer Kamera (140) aufgenommen wird, die so konfiguriert ist, dass sie die anatomische Struktur (120) betrachtet, wobei das anfängliche Kamerabild mit der anatomischen Struktur (120) in der anfänglichen Lage (α₁, ϕ₁) in Bezug auf das Projektionsröntgenbildgebungssystem (130a, 130b) aufgenommen wird; und
ein nachfolgendes Kamerabild (320₂), das die anatomische Struktur (120) darstellt, wobei das nachfolgende Kamerabild von einer Kamera (140) aufgenommen wird, die so konfiguriert ist, dass sie die anatomische Struktur (120) betrachtet, wobei das nachfolgende Kamerabild (320₂) mit der anatomischen Struktur (120) in einer nachfolgenden Lage (α₂, ϕ₂) relativ zum Projektionsröntgenbildgebungssystem (130a, 130b) und zu einem späteren Zeitpunkt (T₂) als das anfängliche Kamerabild (320₁) aufgenommen wird;
Bestimmen (S320), basierend auf dem anfänglichen Röntgenprojektionsbild (310₁), von Lageanpassungsinformationen (110) zum Anpassen der anfänglichen Lage (α₁, ϕ₁) der anatomischen Struktur (120) in Bezug auf das Projektionsröntgenbildgebungssystem (130a, 130b), um das Zielröntgenprojektionsbild (310_{T}) aufzunehmen, das die anatomische Struktur (120) darstellt;
Bestimmen (S330) einer Registrierung (330) zwischen der anatomischen Struktur (120) im anfänglichen Röntgenprojektionsbild (310₁) und der anatomischen Struktur (120) im anfänglichen Kamerabild (320₁);
Verformen (S340) des anfänglichen Kamerabilds (320₁) unter Verwendung der Registrierung (330) und der Lageanpassungsinformationen (110), um ein Zielkamerabild (320_{T}) bereitzustellen, das dem Zielröntgenprojektionsbild (310_{T}) entspricht;
Bestimmen (S350) der Lageanpassungsinformationen (310) zum Anpassen der nachfolgenden Lage (α₂, ϕ₂) der anatomischen Struktur (120) in Bezug auf das Projektionsröntgenbildgebungssystem (130a, 130b), um das Zielröntgenprojektionsbild (310_{T}) aufzunehmen, das die anatomische Struktur (120) darstellt, basierend auf einer Abweichung zwischen dem nachfolgenden Kamerabild (320₂) und dem Zielkamerabild (320_{T}); und
Ausgeben (S360) der Lageanpassungsinformationen (310).

2. Computerimplementiertes Verfahren nach Anspruch 1, wobei die Kamerabilder optische Kamerabilder, die von einer optischen Kamera aufgenommen werden, und/oder Tiefenkamerabilder, die von einer Tiefenkamera aufgenommen werden, umfassen.

3. Computerimplementiertes Verfahren nach Anspruch 1, wobei die Kamera (140) weiter so konfiguriert ist, dass sie mindestens einen Abschnitt des Projektionsröntgenbildgebungssystems (130a, 130b) betrachtet, und wobei das Kamerabild (140) weiter den mindestens einen Abschnitt des Projektionsröntgenbildgebungssystems (130a, 130b) darstellt.

4. Computerimplementiertes Verfahren nach Anspruch 1, wobei das Bestimmen (S320) von Lageanpassungsinformationen (110) zum Anpassen der anfänglichen Lage (α₁, ϕ₁) der anatomischen Struktur (120) in Bezug auf das Projektionsröntgenbildgebungssystem (130a, 130b) Folgendes umfasst:
Eingeben des anfänglichen Röntgenprojektionsbilds (310₁) in ein neuronales Netzwerk; und
wobei das neuronale Netzwerk darauf trainiert ist, die Lageanpassungsinformationen (110) unter Verwendung von Trainingsdaten zu erzeugen, die eine Vielzahl von Trainingsbildern umfassen, die die anatomische Struktur (120) darstellen, wobei die Trainingsbilder Röntgenprojektionsbilder umfassen, die mit der anatomischen Struktur (120) in einer aktuellen Lage in Bezug auf das Projektionsröntgenbildgebungssystem (130a, 130b) aufgenommen wurden, und wobei die Trainingsdaten weiter für jedes Trainingsbild entsprechende Lageanpassungsinformationen umfassen, um die aktuelle Lage der anatomischen Struktur in Bezug auf das Projektionsröntgenbildgebungssystem (130a, 130b) anzupassen, um das Zielröntgenprojektionsbild (310_{T}) aufzunehmen, das die anatomische Struktur (120) darstellt.

5. Computerimplementiertes Verfahren nach einem vorstehenden Anspruch, wobei die anatomische Struktur (120) einen oder mehrere Knochen und das den einen oder die mehreren Knochen umgebende Gewebe umfasst, und wobei das Bestimmen (S330) einer Registrierung (330) zwischen der anatomischen Struktur (120) im anfänglichen Röntgenprojektionsbild (310₁) und der anatomischen Struktur (120) im anfänglichen Kamerabild (320₁) unter Verwendung eines kinematischen Modells durchgeführt wird, das die Knochen und das Gewebe darstellt.

6. Computerimplementiertes Verfahren nach einem vorstehenden Anspruch, wobei die Operation des Empfangens eines nachfolgenden Kamerabilds (320₂), das die anatomische Struktur (120) darstellt, iterativ durchgeführt wird; und wobei in jeder Iteration die folgenden Operationen durchgeführt werden:
Bestimmen (S350) der Lageanpassungsinformationen (310) zum Anpassen der nachfolgenden Lage (α₂, ϕ₂) der anatomischen Struktur (120) in Bezug auf das Projektionsröntgenbildgebungssystem (130a, 130b), um das Zielröntgenprojektionsbild (310_{T}) aufzunehmen, das die anatomische Struktur (120) darstellt, basierend auf einer Abweichung zwischen dem nachfolgenden Kamerabild (320₂) und dem Zielkamerabild (320_{T}); und
Ausgeben (S360) der Lageanpassungsinformationen (310).

7. Computerimplementiertes Verfahren nach Anspruch 1, wobei die Lageanpassungsinformationen (310) zum Anpassen der anfänglichen Lage (α₁,ϕ₁) der anatomischen Struktur (120) in Bezug auf das Projektionsröntgenbildgebungssystem (130a, 130b), um das Zielröntgenprojektionsbild (310_{T}) aufzunehmen, das die anatomische Struktur (120) darstellt, eine Anpassung an eine absolute Position und/oder eine absolute Orientierung der anatomischen Struktur (120) umfasst.

8. Computerimplementiertes Verfahren nach Anspruch 1, wobei die anatomische Struktur (120) eine Vielzahl anatomischer Merkmale (150₁, 150₂) beinhaltet, und wobei die Lageanpassungsinformationen (310) zum Anpassen der anfänglichen Lage (α₁, ϕ₁) der anatomischen Struktur (120) in Bezug auf das Projektionsröntgenbildgebungssystem (130a, 130b) eine Anpassung an eine gegenseitige Positionierung (ϕ) der anatomischen Merkmale darstellen.

9. Computerimplementiertes Verfahren nach Anspruch 1, wobei die Lageanpassungsinformationen (310) in Bezug auf die anfängliche Lage (α₁, ϕ₁) definiert sind, oder wobei die Lageanpassungsinformationen (310) in Bezug auf eine Orientierung einer Mittelachse (160) des Projektionsröntgenbildgebungssystems (130a, 130b) definiert sind.

10. Computerimplementiertes Verfahren nach einem vorstehenden Anspruch, wobei das Ausgeben (S360) der Lageanpassungsinformationen (310) Folgendes umfasst:
Projizieren einer grafischen Darstellung der Lageanpassungsinformationen (310); oder
Ausgeben des empfangenen nachfolgenden Kamerabilds (320₂), das die anatomische Struktur (120) darstellt; und
Ausgeben der Lageanpassungsinformationen (310) als eine Überlagerung auf dem empfangenen nachfolgenden Kamerabild (320₂).

11. Computerimplementiertes Verfahren nach Anspruch 10, wobei das Ausgeben (S360) der Lageanpassungsinformationen (310) als eine Überlagerung auf dem nachfolgenden Kamerabild (320₂) Ausgeben der Lageanpassungsinformationen (310) in einem numerischen Format oder Ausgeben der Lageanpassungsinformationen in einem grafischen Format (310) umfasst.

12. Computerprogrammprodukt, das Anweisungen umfasst, die, wenn sie von einem oder mehreren Prozessoren (210) ausgeführt werden, den einen oder die mehreren Prozessoren (210) veranlassen, ein Verfahren zum Bereitstellen von Lageanpassungsinformationen (310) zum Anpassen einer Lage (α₂, ϕ₂) einer anatomischen Struktur (120) in Bezug auf ein Projektionsröntgenbildgebungssystem (130a, 130b) durchzuführen, um ein Zielröntgenprojektionsbild (310_{T}) aufzunehmen, das die anatomische Struktur (120) darstellt, wobei das Verfahren Folgendes umfasst:
Empfangen (S310) von Bilddaten, wobei die Bilddaten Folgendes umfassen:
ein anfängliches Röntgenprojektionsbild (310₁), das die anatomische Struktur (120) darstellt, wobei das anfängliche Röntgenprojektionsbild vom Projektionsröntgenbildgebungssystem (130a, 130b) mit der anatomischen Struktur (120) in einer anfänglichen Lage (α₁, ϕ₁) in Bezug auf das Projektionsröntgenbildgebungssystem (130a, 130b) aufgenommen wird;
ein anfängliches Kamerabild (320₁), das die anatomische Struktur (120) darstellt, wobei das anfängliche Kamerabild von einer Kamera (140) aufgenommen wird, die so konfiguriert ist, dass sie die anatomische Struktur (120) betrachtet, wobei das anfängliche Kamerabild mit der anatomischen Struktur (120) in der anfänglichen Lage (α₁, ϕ₁) in Bezug auf das Projektionsröntgenbildgebungssystem (130a, 130b) aufgenommen wird; und
ein nachfolgendes Kamerabild (320₂), das die anatomische Struktur (120) darstellt, wobei das nachfolgende Kamerabild von einer Kamera (140) aufgenommen wird, die so konfiguriert ist, dass sie die anatomische Struktur (120) betrachtet, wobei das nachfolgende Kamerabild (320₂) mit der anatomischen Struktur (120) in einer nachfolgenden Lage (α₂, ϕ₂) relativ zum Projektionsröntgenbildgebungssystem (130a, 130b) und zu einem späteren Zeitpunkt (T₂) als das anfängliche Kamerabild (320₁) aufgenommen wird;
Bestimmen (S320), basierend auf dem anfänglichen Röntgenprojektionsbild (310₁), von Lageanpassungsinformationen (110) zum Anpassen der anfänglichen Lage (α₁, ϕ₁) der anatomischen Struktur (120) in Bezug auf das Projektionsröntgenbildgebungssystem (130a, 130b), um das Zielröntgenprojektionsbild (310_{T}) aufzunehmen, das die anatomische Struktur (120) darstellt;
Bestimmen (S330) einer Registrierung (330) zwischen der anatomischen Struktur (120) im anfänglichen Röntgenprojektionsbild (310₁) und der anatomischen Struktur (120) im anfänglichen Kamerabild (320₁);
Verformen (S340) des anfänglichen Kamerabilds (320₁) unter Verwendung der Registrierung (330) und der Lageanpassungsinformationen (110), um ein Zielkamerabild (320_{T}) bereitzustellen, das dem Zielröntgenprojektionsbild (310_{T}) entspricht;
Bestimmen (S350) der Lageanpassungsinformationen (310) zum Anpassen der nachfolgenden Lage (α₂, ϕ₂) der anatomischen Struktur (120) in Bezug auf das Projektionsröntgenbildgebungssystem (130a, 130b), um das Zielröntgenprojektionsbild (310_{T}) aufzunehmen, das die anatomische Struktur (120) darstellt, basierend auf einer Abweichung zwischen dem nachfolgenden Kamerabild (320₂) und dem Zielkamerabild (320_{T}); und
Ausgeben (S360) der Lageanpassungsinformationen (310).

13. System (100) zum Bereitstellen von Lageanpassungsinformationen (310) zum Anpassen einer Lage (α₂, ϕ₂) einer anatomischen Struktur (120) in Bezug auf ein Projektionsröntgenbildgebungssystem (130a, 130b), um ein Zielröntgenprojektionsbild (310_{T}) aufzunehmen, das die anatomische Struktur (120) darstellt, wobei das System, das einen oder mehrere Prozessoren (210) umfasst, die konfiguriert zum:
Empfangen (S310) von Bilddaten, wobei die Bilddaten Folgendes umfassen:
ein anfängliches Röntgenprojektionsbild (310₁), das die anatomische Struktur (120) darstellt, wobei das anfängliche Röntgenprojektionsbild vom Projektionsröntgenbildgebungssystem (130a, 130b) mit der anatomischen Struktur (120) in einer anfänglichen Lage (α₁, ϕ₁) in Bezug auf das Projektionsröntgenbildgebungssystem (130a, 130b) aufgenommen wird;
ein anfängliches Kamerabild (320₁), das die anatomische Struktur (120) darstellt, wobei das anfängliche Kamerabild von einer Kamera (140) aufgenommen wird, die so konfiguriert ist, dass sie die anatomische Struktur (120) betrachtet, wobei das anfängliche Kamerabild mit der anatomischen Struktur (120) in der anfänglichen Lage (α₁, ϕ₁) in Bezug auf das Projektionsröntgenbildgebungssystem (130a, 130b) aufgenommen wird; und
ein nachfolgendes Kamerabild (320₂), das die anatomische Struktur (120) darstellt, wobei das nachfolgende Kamerabild von einer Kamera (140) aufgenommen wird, die so konfiguriert ist, dass sie die anatomische Struktur (120) betrachtet, wobei das nachfolgende Kamerabild (320₂) mit der anatomischen Struktur (120) in einer nachfolgenden Lage (α₂, ϕ₂) relativ zum Projektionsröntgenbildgebungssystem (130a, 130b) und zu einem späteren Zeitpunkt (T₂) als das anfängliche Kamerabild (320₁) aufgenommen wird;
Bestimmen (S320), basierend auf dem anfänglichen Röntgenprojektionsbild (310₁), von Lageanpassungsinformationen (110) zum Anpassen der anfänglichen Lage (α₁, ϕ₁) der anatomischen Struktur (120) in Bezug auf das Projektionsröntgenbildgebungssystem (130a, 130b), um das Zielröntgenprojektionsbild (310_{T}) aufzunehmen, das die anatomische Struktur (120) darstellt;
Bestimmen (S330) einer Registrierung (330) zwischen der anatomischen Struktur (120) im anfänglichen Röntgenprojektionsbild (310₁) und der anatomischen Struktur (120) im anfänglichen Kamerabild (320₁);
Verformen (S340) des anfänglichen Kamerabilds (320₁) unter Verwendung der Registrierung (330) und der Lageanpassungsinformationen (110), um ein Zielkamerabild (320_{T}) bereitzustellen, das dem Zielröntgenprojektionsbild (310_{T}) entspricht;
Bestimmen (S350) der Lageanpassungsinformationen (310) zum Anpassen der nachfolgenden Lage (α₂, ϕ₂) der anatomischen Struktur (120) in Bezug auf das Projektionsröntgenbildgebungssystem (130a, 130b), um das Zielröntgenprojektionsbild (310_{T}) aufzunehmen, das die anatomische Struktur (120) darstellt, basierend auf einer Abweichung zwischen dem nachfolgenden Kamerabild (320₂) und dem Zielkamerabild (320_{T}); und
Ausgeben (S360) der Lageanpassungsinformationen (310).

## Revendications

1. Procédé mis en œuvre par ordinateur de fourniture d'informations d'ajustement de position (310) pour ajuster une position (α₂, ϕ₂) d'une structure anatomique (120) par rapport à un système d'imagerie radiographique par projection ((130a, 130b)) afin d'acquérir une image radiographique par projection cible (310_{T}) représentant la structure anatomique (120), le procédé comprenant :
la réception (S310) de données d'image, les données d'image comprenant :
une image de projection radiographique initiale (310₁) représentant la structure anatomique (120), l'image de projection radiographique initiale étant acquise par le système d'imagerie radiographique par projection (130a, 130b) avec la structure anatomique (120) dans une position initiale (α₁, ϕ₁) par rapport au système d'imagerie radiographique par projection (130a, 130b) ;
une image de caméra initiale (320₁) représentant la structure anatomique (120), l'image de caméra initiale étant acquise par une caméra (140) configurée pour visualiser la structure anatomique (120), l'image de caméra initiale étant acquise avec la structure anatomique (120) dans la position initiale (α₁, ϕ₁) par rapport au système d'imagerie radiographique par projection (130a, 130b) ; et
une image de caméra suivante (320₂) représentant la structure anatomique (120), l'image de caméra suivante étant acquise par une caméra (140) configurée pour visualiser la structure anatomique (120), l'image de caméra suivante (320₂) étant acquise avec la structure anatomique (120) dans une position suivante (α₂, ϕ₂) par rapport au système d'imagerie radiographique par projection (130a, 130b) et à un instant ultérieur (T₂) à celui de l'image de caméra initiale (320₁) ;
la détermination (S320), basée sur l'image de projection radiographique initiale (310₁), d'informations d'ajustement de position (110) pour ajuster la position initiale (α₁, ϕ₁) de la structure anatomique (120) par rapport au système d'imagerie radiographique par projection (130a, 130b) afin d'acquérir l'image de projection radiographique cible (310_{T}) représentant la structure anatomique (120) ;
la détermination (S330) d'un alignement (330) entre la structure anatomique (120) dans l'image de projection radiographique initiale (310₁) et la structure anatomique (120) dans l'image de caméra initiale (320₁) ;
la déformation (S340) de l'image de caméra initiale (320₁) à l'aide de l'alignement (330) et des informations d'ajustement de position (110) pour fournir une image de caméra cible, (320_{T}) correspondant à l'image de projection radiographique cible (310_{T}) ;
la détermination (S350) des informations d'ajustement de position (310) pour ajuster la position suivante (α₂, ϕ₂) de la structure anatomique (120) par rapport au système d'imagerie radiographique par projection (130a, 130b) afin d'acquérir l'image radiographique par projection cible (310_{T}) représentant la structure anatomique (120), sur la base d'un écart entre l'image de caméra suivante (320₂) et l'image de caméra cible (320₁) ; et
la production en sortie (S360) des informations d'ajustement de position (310).

2. Procédé mis en œuvre par ordinateur selon la revendication 1, dans lequel les images de caméra comprennent des images de caméra optique acquises par une caméra optique et/ou des images de caméra de profondeur acquises par une caméra de profondeur.

3. Procédé mis en œuvre par ordinateur selon la revendication 1, dans lequel la caméra (140) est en outre configurée pour visualiser au moins une partie du système d'imagerie radiographique par projection (130a, 130b), et dans lequel l'image de caméra (140) représente en outre la au moins une partie du système d'imagerie radiographique par projection (130a, 130b).

4. Procédé mis en œuvre par ordinateur selon la revendication 1, dans lequel la détermination (S320) d'informations d'ajustement de position (110) pour ajuster la position initiale (α₁, ϕ₁) de la structure anatomique (120) par rapport au système d'imagerie radiographique par projection (130a, 130b), comprend :
l'entrée de l'image de projection radiographique initiale (310₁) dans un réseau neuronal ; et
dans lequel le réseau neuronal est entraîné à générer les informations d'ajustement de position (110) à l'aide de données d'entraînement comprenant une pluralité d'images d'entraînement représentant la structure anatomique (120), les images d'entraînement comprenant des images de projection radiographique acquises avec la structure anatomique (120) dans une position actuelle par rapport au système d'imagerie radiographique par projection (130a, 130b), et dans lequel les données d'entraînement comprennent en outre, pour chaque image d'entraînement, des informations d'ajustement de position correspondantes pour ajuster la position actuelle de la structure anatomique par rapport au système d'imagerie radiographique par projection (130a, 130b) afin d'acquérir l'image de projection radiographique cible (310_{T}) représentant la structure anatomique (120).

5. Procédé mis en œuvre par ordinateur selon une quelconque revendication précédente, dans lequel la structure anatomique (120) comprend un ou plusieurs os et des tissus entourant les un ou plusieurs os, et dans lequel la détermination (S330) d'un alignement (330) entre la structure anatomique (120) dans l'image de projection radiographique initiale (310₁) et la structure anatomique (120) dans l'image de caméra initiale (320₁), est effectuée à l'aide d'un modèle cinématique représentant les os et les tissus.

6. Procédé mis en œuvre par ordinateur selon une quelconque revendication précédente, dans lequel l'opération de réception d'une image de caméra suivante (320₂) représentant la structure anatomique (120) est effectuée de manière itérative ; et dans lequel les opérations suivantes sont effectuées à chaque itération :
la détermination (S350) des informations d'ajustement de position (310) pour ajuster la position suivante (α₂, ϕ₂) de la structure anatomique (120) par rapport au système d'imagerie radiographique par projection (130a, 130b) afin d'acquérir l'image radiographique par projection cible (310_{T}) représentant la structure anatomique (120), sur la base d'un écart entre l'image de caméra suivante (320₂) et l'image de caméra cible (320₁) ; et
la production en sortie (S360) des informations d'ajustement de position (310).

7. Procédé mis en œuvre par ordinateur selon la revendication 1, dans lequel les informations d'ajustement de position (310) permettant d'ajuster la position initiale (α₁, ϕ₁) de la structure anatomique (120) par rapport au système d'imagerie radiographique par projection (130a, 130b) afin d'acquérir l'image radiographique par projection cible (310_{T}) représentant la structure anatomique (120), comprennent un ajustement d'une position absolue et/ou d'une orientation absolue de la structure anatomique (120).

8. Procédé mis en œuvre par ordinateur selon la revendication 1, dans lequel la structure anatomique (120) inclut une pluralité de caractéristiques anatomiques (150₁, 150₂), et dans lequel les informations d'ajustement de position (310) pour ajuster la position initiale (α₁, ϕ₁) de la structure anatomique (120) par rapport au système d'imagerie radiographique par projection (130a, 130b) représentent un ajustement d'un positionnement mutuel (ϕ) des caractéristiques anatomiques.

9. Procédé mis en œuvre par ordinateur selon la revendication 1, dans lequel les informations d'ajustement de position (310) sont définies par rapport à la position initiale (α₁, ϕ₁), ou dans lequel les informations d'ajustement de position (310) sont définies par rapport à une orientation d'un axe central (160) du système d'imagerie radiographique par projection (130a, 130b).

10. Procédé mis en œuvre par ordinateur selon une quelconque revendication précédente, dans lequel la production en sortie (S360) des informations d'ajustement de position (310) comprend :
la projection d'une représentation graphique des informations d'ajustement de position (310) ; ou
la production en sortie de l'image de caméra suivante (320₂) reçue représentant la structure anatomique (120) ; et
la production en sortie des informations d'ajustement de position (310) en superposition sur l'image de caméra suivante (320₂) reçue.

11. Procédé mis en œuvre par ordinateur selon la revendication 10, dans lequel la production en sortie (S360) des informations d'ajustement de position (310) en superposition sur l'image de caméra suivante (320₂), comprend la production en sortie des informations d'ajustement de position (310) dans un format numérique, ou la production en sortie des informations d'ajustement de position dans un format graphique (310).

12. Produit de programme informatique comprenant des instructions qui, lorsqu'elles sont exécutées par un ou plusieurs processeurs (210), amènent les un ou plusieurs processeurs (210) à mettre en œuvre un procédé de fourniture d'informations d'ajustement de position (310) pour ajuster une position (α₂, ϕ₂) d'une structure anatomique (120) par rapport à un système d'imagerie radiographique par projection (130a, 130b) afin d'acquérir une image radiographique par projection cible (310_{T}) représentant la structure anatomique (120), le procédé comprenant :
la réception (S310) de données d'image, les données d'image comprenant :
une image de projection radiographique initiale (310₁) représentant la structure anatomique (120), l'image de projection radiographique initiale étant acquise par le système d'imagerie radiographique par projection (130a, 130b) avec la structure anatomique (120) dans une position initiale (α₁, ϕ₁) par rapport au système d'imagerie radiographique par projection (130a, 130b) ;
une image de caméra initiale (320₁) représentant la structure anatomique (120), l'image de caméra initiale étant acquise par une caméra (140) configurée pour visualiser la structure anatomique (120), l'image de caméra initiale étant acquise avec la structure anatomique (120) dans la position initiale (α₁, ϕ₁) par rapport au système d'imagerie radiographique par projection (130a, 130b) ; et
une image de caméra suivante (320₂) représentant la structure anatomique (120), l'image de caméra suivante étant acquise par une caméra (140) configurée pour visualiser la structure anatomique (120), l'image de caméra suivante (320₂) étant acquise avec la structure anatomique (120) dans une position suivante (α₂, ϕ₂) par rapport au système d'imagerie radiographique par projection (130a, 130b) et à un instant ultérieur (T₂) à celui de l'image de caméra initiale (320₁) ;
la détermination (S320), sur la base de l'image de projection radiographique initiale (310₁), des informations d'ajustement de position (110) pour ajuster la position initiale (α₁, ϕ₁) de la structure anatomique (120) par rapport au système d'imagerie radiographique par projection (130a, 130b) afin d'acquérir l'image de projection radiographique cible (310_{T}) représentant la structure anatomique (120) ;
la détermination (S330) d'un alignement (330) entre la structure anatomique (120) dans l'image de projection radiographique initiale (310₁) et la structure anatomique (120) dans l'image de caméra initiale (320₁) ;
la déformation (S340) de l'image de caméra initiale (320₁) à l'aide de l'alignement (330) et des informations d'ajustement de position (110) pour fournir une image de caméra cible (320₁), correspondant à l'image de projection radiographique cible (310_{T}) ;
la détermination (S350) des informations d'ajustement de position (310) pour ajuster la position suivante (α₂, ϕ₂) de la structure anatomique (120) par rapport au système d'imagerie radiographique par projection (130a, 130b) afin d'acquérir l'image radiographique par projection cible (310_{T}) représentant la structure anatomique (120), sur la base d'un écart entre l'image de caméra suivante (320₂) et l'image de caméra cible (320₁) ; et
la production en sortie (S360) des informations d'ajustement de position (310).

13. Système (100) pour fournir des informations d'ajustement de position (310) pour ajuster une position (α₂, ϕ₂) d'une structure anatomique (120) par rapport à un système d'imagerie radiographique par projection (130a, 130b) afin d'acquérir une image radiographique par projection cible (310_{T}) représentant la structure anatomique (120), le système (100) comprenant un ou plusieurs processeurs (210) configurés pour :
recevoir (S310) des données d'image, les données d'image comprenant :
une image de projection radiographique initiale (310₁) représentant la structure anatomique (120), l'image de projection radiographique initiale étant acquise par le système d'imagerie radiographique par projection (130a, 130b) avec la structure anatomique (120) dans une position initiale (α₁, ϕ₁) par rapport au système d'imagerie radiographique par projection (130a, 130b) ;
une image de caméra initiale (320₁) représentant la structure anatomique (120), l'image de caméra initiale étant acquise par une caméra (140) configurée pour visualiser la structure anatomique (120), l'image de caméra initiale étant acquise avec la structure anatomique (120) dans la position initiale (α₁, ϕ₁) par rapport au système d'imagerie radiographique par projection (130a, 130b) ; et
une image de caméra suivante (320₂) représentant la structure anatomique (120), l'image de caméra suivante étant acquise par une caméra (140) configurée pour visualiser la structure anatomique (120), l'image de caméra suivante (320₂) étant acquise avec la structure anatomique (120) dans une position suivante (α₂, ϕ₂) par rapport au système d'imagerie radiographique par projection (130a, 130b) et à un instant ultérieur (T₂) à celui de l'image de caméra initiale (320₁) ;
déterminer (S320), sur la base de l'image de projection radiographique initiale (310₁), les informations d'ajustement de position (110) pour ajuster la position initiale (α₁, ϕ₁) de la structure anatomique (120) par rapport au système d'imagerie radiographique par projection (130a, 130b) afin d'acquérir l'image de projection radiographique cible (310_{T}) représentant la structure anatomique (120) ;
déterminer (S330) un alignement (330) entre la structure anatomique (120) dans l'image de projection radiographique initiale (310₁) et la structure anatomique (120) dans l'image de caméra initiale (320₁) ;
déformer (S340) l'image de caméra initiale (320₁) à l'aide de l'alignement (330) et des informations d'ajustement de position (110) pour fournir une image de caméra cible, (320_{T}) correspondant à l'image de projection radiographique cible (310_{T}) ;
déterminer (S350) les informations d'ajustement de position (310) pour ajuster la position suivante (α₂, ϕ₂) de la structure anatomique (120) par rapport au système d'imagerie radiographique par projection (130a, 130b) afin d'acquérir l'image radiographique par projection cible (310_{T}) représentant la structure anatomique (120), sur la base d'un écart entre l'image de caméra suivante (320₂) et l'image de caméra cible (320₁) ; et
produire en sortie (S360) les informations d'ajustement de position (310).
